# EUROPEAN PATENT SPECIFICATION

(11) **EP 4 322 835 B1**
(45) Date of publication and mention of the grant of the patent: **04.11.2026**
(21) Application number: 22787171.2
(22) Date of filing: 14.04.2022
(51) Int. Cl.: A61B 5/00, A61B 5/263, A61B 5/30, A61B 5/301, A61N 1/05, A61N 1/36, A61B 5/291, A61B 5/377

(54) **METHODS AND APPARATUS FOR IMPROVED MEASUREMENT OF COMPOUND ACTION POTENTIALS**
VERFAHREN UND VORRICHTUNG ZUR VERBESSERTEN MESSUNG VON VERBINDUNGSAKTIONSPOTENTIALEN
PROCÉDÉS ET APPAREIL POUR UNE MESURE AMÉLIORÉE DE POTENTIELS D'ACTION DE COMPOSÉS

(30) Priority: 16.04.2021 US 202163176005 P; 18.11.2021 AU 2021903719
(43) Date of publication of application: 21.02.2024
(73) Proprietor: Saluda Medical Pty Ltd, Macquarie Park, NSW 2113 (AU)
(72) Inventor: SINGLE, Peter Scott Vallack, Artarmon, NSW 2064 (AU); SCOTT, Jonathan Brereton, Artarmon, NSW 2785 (AU); OLECKO, Joanne Carol Shiels, Pleasanton, California 94566 (US)
(74) Representative: Abel & Imray LLP
(86) International application number: PCT/AU2022/050347
(87) International publication number: WO 2022/217322

(56) References cited:
- WO-A1-2020/082126
- WO-A1-2020/109261
- US-A1- 2015 282 725
- US-A1- 2019 183 376
- US-A1- 2019 357 788
- P.L. TABERNA ; C. PORTET ; P. SIMON: "Electrode surface treatment and electrochemical impedance spectroscopy study on carbon/carbon supercapacitors", APPLIED PHYSICS A; MATERIALS SCIENCE & PROCESSING, SPRINGER, BERLIN, DE, vol. 82, no. 4, 1 March 2006 (2006-03-01), Berlin, DE , pages 639 - 646, XP019337179, ISSN: 1432-0630, DOI: 10.1007/s00339-005-3404-0

## Description

### Technical Field

The present invention relates to measurement of compound action potentials evoked by neurostimulation. In particular, the invention relates to reducing or removing the effect of stimulus artefact generated by measurement circuitry and measurement electrode kinematics, to facilitate measurement of compound action potentials.

### Background of the Invention

There are a range of situations in which it is desirable to apply neural stimuli in order to give rise to an evoked compound action potential (ECAP). For example, neuromodulation is used to treat a variety of disorders including chronic pain, Parkinson's disease, and migraine. A neuromodulation system applies an electrical pulse to tissue in order to generate a therapeutic effect. When used to relieve chronic pain, the electrical pulse is applied to the dorsal column (DC) of the spinal cord, referred to as spinal cord stimulation (SCS). Neuromodulation systems typically comprise an implanted electrical pulse generator, and a power source such as a battery that may be rechargeable by transcutaneous inductive transfer. An electrode array is connected to the pulse generator, and is positioned adjacent the nerve of interest such as in the dorsal epidural space above the dorsal column. An electrical pulse applied to the dorsal column by an electrode causes the depolarisation of neurons, and the generation of propagating action potentials. The fibres being stimulated in this way inhibit the transmission of pain from that segment in the spinal cord to the brain. To sustain the pain relief effects, stimuli are applied substantially continuously, for example at a frequency in the range of 50-100 Hz. WO2020/082126 discloses a device arranged to minimise artefacts in neurostimulation devices. US2019/357788 discloses an apparatus and method for measuring a neural response to a stimulus. US2019/183376 discloses a biopotential measuring apparatus with a stimulating section for stimulating a living body. US2015/282725 discloses a method and apparatus for controlling electrical conditions of tissue in relation to a current stimulus.

Neuromodulation may also be used to stimulate efferent fibres, for example to induce motor functions. In general, the electrical stimulus generated in a neuromodulation system triggers one or more neural action potentials, which then have either an inhibitory or excitatory effect. Inhibitory effects can be used to modulate an undesired process such as the transmission of pain, or excitatory effects may for example cause a desired effect such as a contraction of a muscle.

There are a range of circumstances in which it is desirable to obtain an electrical measurement of an ECAP evoked on a neural pathway by an electrical stimulus applied to the neural pathway. However, this can be a difficult task as an observed ECAP signal will typically have a maximum amplitude of a few tens of microvolts or less, whereas a stimulus applied to evoke the ECAP is typically several volts. Stimulus artefact usually results from the stimulus, and manifests as a decaying output of several millivolts or hundreds of microvolts throughout the time that the ECAP occurs, presenting a significant obstacle to isolating the much smaller ECAP of interest. As the neural response can be contemporaneous with the stimulus and/or the stimulus artefact, ECAP measurements present a difficult challenge of implant design. In practice, many non-ideal aspects of a circuit lead to artefact, and as these mostly have a decaying exponential characteristic which can be of either positive or negative polarity, identification and elimination of sources of artefact can be laborious. A number of approaches have been proposed for recording an ECAP, including those of King (US Patent No. 5,913,882), Nygard (US Patent No. 5,758,651), Daly (US Patent Application No. 2007/0225767) and the present Applicant (US Patent No. 9,386,934).

Evoked responses are less difficult to detect when they appear after the artefact has decayed, or when the signal-to-noise ratio is sufficiently high. The period of high artefact is often restricted to a time of 1 - 2 ms after the stimulus and so, provided the neural response is detected after this time window, data can be obtained. This is the case in surgical monitoring where there are large distances between the stimulating and recording electrodes so that the neural response propagation time from the stimulus site to the recording electrodes exceeds 2 ms. However, neurostimulation implants are by necessity compact devices. To characterize responses evoked by a single implant such as responses from the dorsal columns to SCS, for example, high stimulation currents and close proximity between electrodes are required, and therefore the measurement process must overcome contemporaneous stimulus artefact directly, greatly exacerbating the difficulty of neural measurement.

Similar considerations can arise in deep brain stimulation where it can be desirable to stimulate a neural structure and immediately measure the evoked compound action potential produced in that structure before the neural response propagates elsewhere in the brain. Artefact remains a significant obstacle to measurement of neural responses proximal to the stimulus location, with the consequence that most neurostimulation implants do not take any measurements whatsoever of neural responses evoked by the implant's stimuli.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

In this specification, a statement that an element may be "at least one of" a list of options is to be understood that the element may be any one of the listed options, or may be any combination of two or more of the listed options.

### Summary of the Invention

According to a first aspect the present invention provides a device for recording evoked neural responses, the device comprising:
a plurality of electrodes including one or more stimulus electrodes, a first sense electrode, and a second sense electrode;
a stimulus source for providing a stimulus to be delivered from the one or more stimulus electrodes to a neural pathway in order to give rise to an evoked action potential on the neural pathway;
measurement circuitry for recording a neural compound action potential signal sensed at the first sense electrode and second sense electrode; and characterised by
impedance compensation means configured to compensate for an impedance difference, the impedance difference being a difference between an impedance associated with the first sense electrode and an impedance associated with the second sense electrode.

The impedance compensation means may improve performance of detection of evoked compound action potentials (ECAPs) and closed loop feedback control of spinal cord stimulation (SCS).

According to a second aspect the present invention provides a method for recording evoked neural responses, the method comprising:
delivering, by a stimulus source, a stimulus via one or more stimulus electrodes of a plurality of electrodes to a neural pathway in order to evoke a compound action potential on the neural pathway;
processing, with measurement circuitry, a signal sensed at a first sense electrode and a second sense electrode of the plurality of electrodes, the sensed signal comprising the evoked compound action potential; and characterised by
compensating for an impedance difference, the impedance difference being a difference between an impedance associated with the first sense electrode and an impedance associated with the second sense electrode.

In some embodiments the impedance compensation means may comprise one or more compensating impedances connected in a manner to balance the impedance difference. The one or more compensating impedances may have fixed impedance values. Preferably the one or more compensating impedances have configurable impedance values, which may be adjusted over time in response to changes in the impedance difference. The configurable impedance values may be adjusted under control of a feedback loop operating based on artefact measured in the sensed signal.

In some embodiments the impedance compensation means may comprise an electrical shield positioned around or substantially around at least one conductor conveying a potential from a sense electrode to the measurement circuitry. In such embodiment the electrical shield may be electrically driven in a manner to reduce artefact. For example the at least one electrical shield may be driven with a voltage derived from one or more unused electrodes of the plurality of electrodes in contact with the body tissue.

In some embodiments the impedance compensation means may comprise an isolator. For example, an implantable pulse generator may comprise:
- an amplifier module configured to amplify the evoked compound action potential to thereby generate a feedback signal;
- a control module configured to receive the feedback signal and to send a control signal to a stimulus source;
- an isolator configured to electrically isolate the measurement circuitry from the control module.

The implantable pulse generator may further comprise a power source configured to provide power to one or more of the amplifier module, control module, and stimulus source. The impedance compensation means may comprise an isolator configured to electrically isolate the power source from the amplifier module.

In some embodiments the isolator may comprise a DC-to-DC convertor configured to convert DC power from the power source to DC power for the amplifier module. In some embodiments a first electrical ground(s) of the power source, control module and stimulus source is independent of a second electrical ground of the amplifier module. In some embodiments an amplifier input impedance, from the sense electrodes to the second electrical ground, is in the order of 200 megohms of resistance and less than 10 pF of stray capacitance. In some embodiments the first electrical ground is grounded to a case housing the implantable pulse generator.

In some embodiments the isolator comprises an opto-isolator configured to electrically isolate the amplifier module from the control module. In some embodiments the isolator is configured such that a coupling capacitance, between the amplifier module and the control module, is lower than or equal to 20 pF. In some embodiments the coupling capacitance is in the range of 2 to 20pF.

In some embodiments the impedance compensation means comprises an input capacitance of the measurement circuitry that is small enough that artefact induced by the impedance difference in the sensed signal is below a predetermined limit at a predetermined time after the delivered stimulus.

In some embodiments the impedance compensation means may comprise a surface treatment to one or both of the sense electrodes, wherein the or each surface treatment is configured to reduce the impedance associated with the corresponding sense electrode.

The surface treatment may be a coating configured to increase the polarizability of the sense electrode.

The surface treatment may be configured to increase the surface area of the electrode. The surface treatment may comprise a grooving or a roughening.

The surface treatment may comprise minimising a length of the electrode that is exposed to a stimulation field, for example by enclosing the electrode in an insulator with a small opening in the insulator.

In some embodiments a method of closed-loop spinal cord stimulation with an implantable pulse generator comprises:
- delivering a stimulation current with a stimulator module to one or more one or more stimulation leads;
- amplifying, with an amplifier module, an evoked compound action potential received at one or more sense electrodes to generate a feedback signal;
- sending the feedback signal, from the amplifier module to a control module, via an isolator to electrically isolate the feedback signal between the amplifier module and the control module;
- determining, by the control module, a control signal based, at least in part, on the feedback signal; and
- sending the control signal from the control module to the stimulator module to deliver further stimulation current to the one or more stimulation leads.

In some embodiments this method further comprises sending power from a power source to the amplifier module via the isolator, or another isolator, to electrically isolate the power source and the amplifier module. In some embodiments the method further comprises grounding the power source, control module and stimulator module to a first electrical ground(s); and grounding the amplifier module to a second electrical ground that is independent of the first electrical ground(s). In some embodiments the step of sending the feedback signal via an isolator comprises an opto-isolator converting the feedback signal from the amplifier module in the form of an electrical signal to an optical signal; and converting the optical signal from the optical signal to another electrical signal as the feedback signal to the control module.

According to an example embodiment not covered by the present claims, there is provideda device for recording evoked neural responses, the device comprising:
a plurality of electrodes including one or more stimulus electrodes and one or more sense electrodes;
a stimulus source for providing a stimulus to be delivered via the one or more stimulus electrodes to a neural pathway in order to evoke a compound action potential on the neural pathway; and
measurement circuitry for processing a signal sensed at the one or more sense electrodes, the sensed signal comprising the evoked compound action potential signal;
wherein at least one of the sense electrodes is provided with a surface treatment, wherein the or each surface treatment is configured to reduce an impedance associated with the corresponding sense electrode.

According to another example embodiment not covered by the present claims, there is provided a method for recording evoked neural responses, the method comprising:
Delivering, by a stimulus source, a stimulus via one or more stimulus electrodes of a plurality of electrodes to a neural pathway in order to evoke a compound action potential on the neural pathway; and
processing, with measurement circuitry, a signal sensed at one or more sense electrodes of the plurality of electrodes, the sensed signal comprising the evoked compound action potential signal;
wherein at least one of the sense electrodes is provided with a surface treatment, and wherein the or each surface treatment is configured to reduce an impedance associated with the corresponding sense electrode.

In some embodiments, the surface treatment may be configured to improve polarizability of the sense electrode. For example, the sense electrode(s) may be provided with a surface treatment such as a titanium-nitride and/or carbon coating, and/or may be treated in a manner to increase a surface area of the electrodes such as by grooving or roughening the electrode surface. Reducing a magnitude of the impedances of each sense electrode will usually reduce a magnitude of a difference between the impedances, and thus reduce the artefact caused by such a difference.

In some embodiments, a clinician or automated test system may review the respective impedances of a plurality of electrodes or all recording electrodes, and then choose two electrodes with the same or similar impedance for use as the sense electrodes.

References herein to estimation or determination are to be understood as referring to an automated process carried out on data by a processor operating to execute a predefined estimation or determination procedure. The approaches presented herein may be implemented in hardware (e.g., using application specific integrated circuits (ASICS)), or in software (e.g., using instructions tangibly stored on computer-readable media for causing a data processing system to perform the steps described above), or in a combination of hardware and software. The invention can also be embodied as computer-readable code on a computer-readable medium. The computer-readable medium can include any data storage device that can store data which can thereafter be read by a computer system. Examples of the computer readable medium include read-only memory ("ROM"), random-access memory ("RAM"), CD-ROMs, DVDs, magnetic tape, optical data storage device, flash storage devices, or any other suitable storage devices. The computer-readable medium can also be distributed over network coupled computer systems so that the computer readable code is stored and executed in a distributed fashion.

In some embodiments the device is an implantable device.

According to a further example embodiment not covered by the present claims there is provided a non-transitory computer readable medium for performing the method of the second aspect, comprising instructions which, when executed by one or more processors, causes performance of the said steps.

### Brief Description of the Drawings

An example of the invention will now be described with reference to the accompanying drawings, in which:
Fig. 1 schematically illustrates an implanted spinal cord stimulator in accordance with the aspects of the present technology;
Fig. 2 is a block diagram of the implanted neurostimulator of Fig. 1;
Fig. 3 is a schematic illustrating interaction of the implanted stimulator with a nerve;
Fig. 4a depicts an example configuration of stimulation and recording electrodes for ECAP recording, and Fig. 4b depicts an electrical model of the configuration of Fig. 4a;
Fig. 5 illustrates stimulus waveforms seen on recording electrodes;
Fig. 6 is a simplified circuit model of the electrical configuration of Fig. 4a;
Fig. 7 is a plot of artefact observed in human patients, relative to absolute electrode impedance; and relative to the impedance difference;
Fig. 8 illustrates H-bridge stimulation;
Fig. 9 illustrates the effect of mismatch impedance on input voltage at the amplifier;
Fig. 10 illustrates resistive and CPE parts of an electrode's impedance in response to a step;
Fig. 11 is a log-log plot of the relationship between the resistive and CPE components;
Fig. 12 illustrates resistive and CPE parts of an electrode's impedance in response to a series of steps;
Fig. 13 is a plot illustrating a relationship of CPE impedance to the impedance difference of the sense electrodes;
Fig. 14 illustrates an aspect of the present technology providing separately adjustable input impedance components;
Fig. 15 is a plot of artefact for electrodes of differing impedance;
Fig. 16 illustrates another aspect of the present technology providing a shield around the conductors conveying the electrode potentials to the amplifier;
Fig. 17 illustrates the reduction in artefact afforded by the implementation of Fig. 16;
Fig. 18 illustrates another implementation providing a multiplexor which selectively drives the shield;
Fig. 19 illustrates a further implementation providing a buffer amplifier to drive the shield;
Fig. 20 is a plot of the reduction in artefact effected by the implementation of Fig. 19;
Fig. 21 illustrates yet another implementation comprising a SCS stimulator having a star-connected capacitor array;
Fig. 22 is a plot of the reduction in artefact effected by the implementation of Fig. 21;
Fig. 23 illustrates selection of bias resistors and a low noise amplifier;
Figs. 24, 25a and 25b depict an implantable pulse generator (IPG) system in accordance with yet a further aspect of the present technology;
Figs. 26 and 27 illustrate an existing IPG system;
Fig. 28 depicts a method in accordance with yet a further aspect of the present technology;
Fig. 29 illustrates reduction of the impedances to pure capacitances;
Fig. 30 illustrates an electrode with low impedance and small effective length in accordance with yet another aspect of the present technology; and
Fig. 31 illustrates an alternative electrode with low impedance and small effective length.

### Description of the Preferred Embodiments

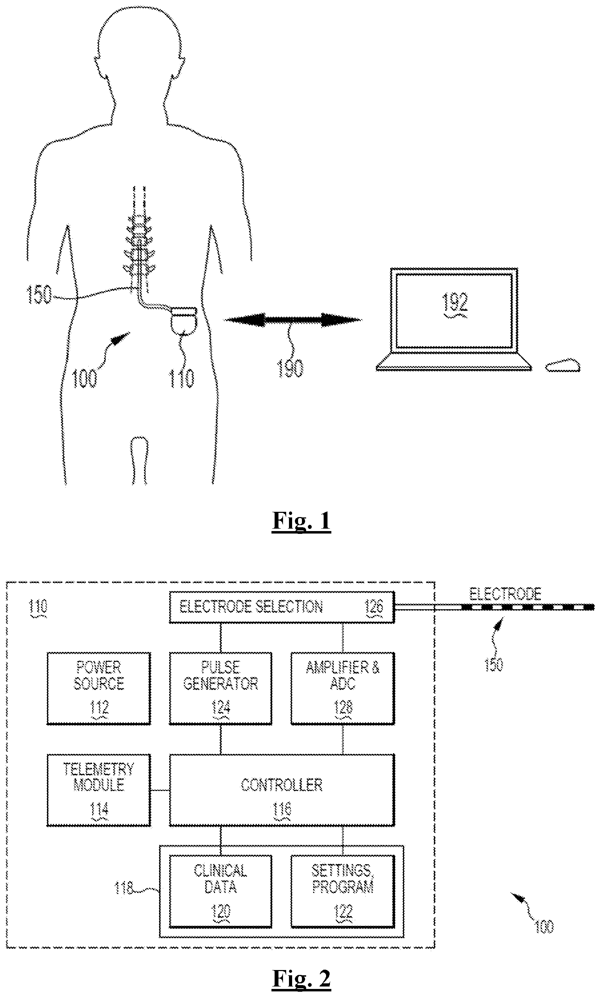

**Fig. 1** schematically illustrates an implanted spinal cord stimulator 100. Stimulator 100 comprises an electronics module 110 implanted at a suitable location in the patient's lower abdominal area or posterior superior gluteal region, and an electrode assembly 150 implanted within the epidural space and connected to the module 110 by a suitable lead. Numerous aspects of operation of implanted neural device 100 are reconfigurable by an external control device 192. Moreover, implanted neural device 100 serves a data gathering role, with gathered data being communicated to external device 192 via any suitable transcutaneous communications channel 190.

**Fig. 2** is a block diagram of the implanted neurostimulator 100. Module 110 contains a battery 112 and a telemetry module 114. In embodiments of the present invention, any suitable type of transcutaneous communication 190, such as infrared (IR), electromagnetic, capacitive and inductive transfer, may be used by telemetry module 114 to transfer power and/or data between an external device 192 and the electronics module 110. Module controller 116 has an associated memory 118 storing patient settings 120, control programs 122 and the like. Controller 116 controls a pulse generator 124 to generate stimuli in the form of current pulses in accordance with the patient settings 120 and control programs 122. Electrode selection module 126 switches the generated pulses to the appropriate electrode(s) of electrode array 150, for delivery of the current pulse to the tissue surrounding the selected electrode(s). Measurement circuitry 128 is configured to capture measurements of neural responses sensed at sense electrode(s) of the electrode array as selected by electrode selection module 126.

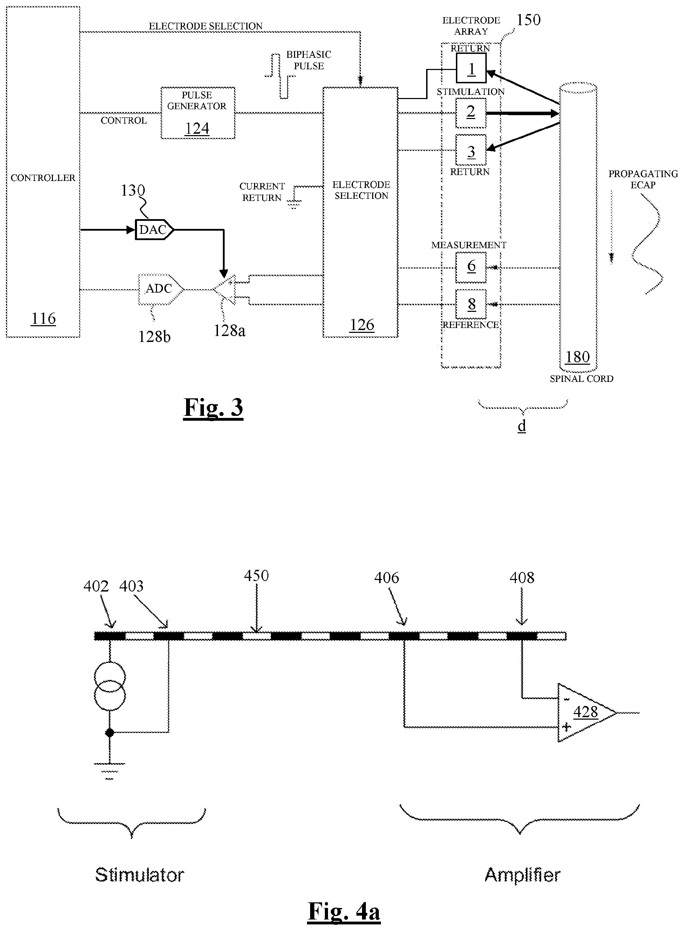

**Fig. 3** is a schematic illustrating interaction of the implanted stimulator 100 with a nerve 180, in this case the spinal cord however alternative embodiments may be positioned adjacent any desired neural tissue including a peripheral nerve, visceral nerve, parasympathetic nerve or a brain structure. The pulse generator 124 produces a suitable stimulus pulse, which in Fig. 3 is shown as a biphasic pulse, although alternative embodiments of the invention may utilise a triphasic pulse or other multiphasic pulse for example in accordance with the teachings of in International Patent Publication No. WO 2017/219096 by the present applicant. Electrode selection module 126 selects a stimulation electrode 2 of electrode array 150 to deliver the electrical current pulse to surrounding tissue including nerve 180, and selects return electrodes 1 and 3 for stimulus current recovery to maintain a zero net charge transfer. In this manner electrode selection module 126 effects tripolar stimulation via stimulus electrodes 1, 2, 3, for example in accordance with the teachings of the above-noted WO 2017/219096 , and/or in accordance with the teachings of International Patent Publication No. WO 2020/082118 by the present applicant. Alternative embodiments may utilise bipolar stimulation by use of two electrodes.

Delivery of an appropriate stimulus to the nerve 180 evokes a neural response comprising a compound action potential which will propagate along the nerve 180 as illustrated, for therapeutic purposes which in the case of a spinal cord stimulator for chronic pain might be to create paraesthesia at a desired location. To this end the stimulus electrodes are used to deliver stimuli at any therapeutically suitable frequency, for example 30 Hz, although other frequencies may be used including as high as the kHz range, and/or stimuli may be delivered in a non-periodic manner such as in bursts, or sporadically, as appropriate for the patient. To fit the device, a clinician typically applies stimuli of various configurations which seek to produce a sensation that is experienced by the user as a paraesthesia, or generally to provide a desirable therapy. When a stimulus configuration is found which evokes paraesthesia, which is in a location and of a size which is congruent with the area of the user's body affected by pain, the clinician nominates that configuration for ongoing use.

The device 100 is further configured to sense the existence and intensity of compound action potentials (CAPs) propagating along nerve 180, whether such CAPs are evoked by the stimulus from electrodes 2 and 4, or otherwise evoked. To this end, any electrodes of the array 150 may be selected by the electrode selection module 126 to serve as measurement electrode 6 and measurement reference electrode 8. Signals sensed by the measurement electrodes (also referred to herein as sense electrodes or recording electrodes) 6 and 8 are passed to measurement circuitry comprising one or more amplifiers 128a, which for example may operate in accordance with the teachings of International Patent Application Publication No. WO2012155183 by the present applicant. The output of the amplifier(s) 128a is then digitised by analog to digital converter 128b and passed to the controller 116. Nevertheless, artefact remains a significant obstacle to measurement of neural responses proximal to the stimulus location. The present Applicant has previously presented a model of the neurostimulation environment, in International Patent Publication No. WO 2020/0821 26.

Recording evoked compound action potentials thus requires the delivery of an electrical stimulus, and the recording of electrical potentials produced by the stimulated nerves. This is challenging because the evoked potentials can be much smaller than the stimuli, for example around six orders of magnitude smaller. Unless special measures are taken, the stimulus obscures the response. For example, in spinal cord stimulation, where a distance *d* between the electrode array 150 and the nerve 180 can be several millimetres, a therapeutically optimal stimulus applied by electrodes 1, 2, 3 can be on the order of 10 volts, while the evoked potential observed on the sense electrodes 6, 8 can be on the order of 10 microvolts. The evoked responses generally must be recorded very quickly after the stimulus, as the duration of the evoked responses is typically quite short, the recording electrodes 6, 8 are close to the stimulus electrodes 1, 2, 3 due to the limited size of the implanted device, and the conduction velocity of the nerve 180 is quite high (e.g. in the range 15-70 m.s⁻¹). As a result, depending on the electrode configuration and the conduction velocity of the nerves stimulated, a 1 millisecond duration of evoked responses is typical. Building a system to directly digitise a waveform with this dynamic range is impractical; in this example, resolving the ECAP to just 4 bits of resolution would require a signal chain and ADC with no less than 24 bits of effective resolution, sampling on the order of 1 kHz. This is not practical with present technology, particularly for a compact implantable device with limited power budget.

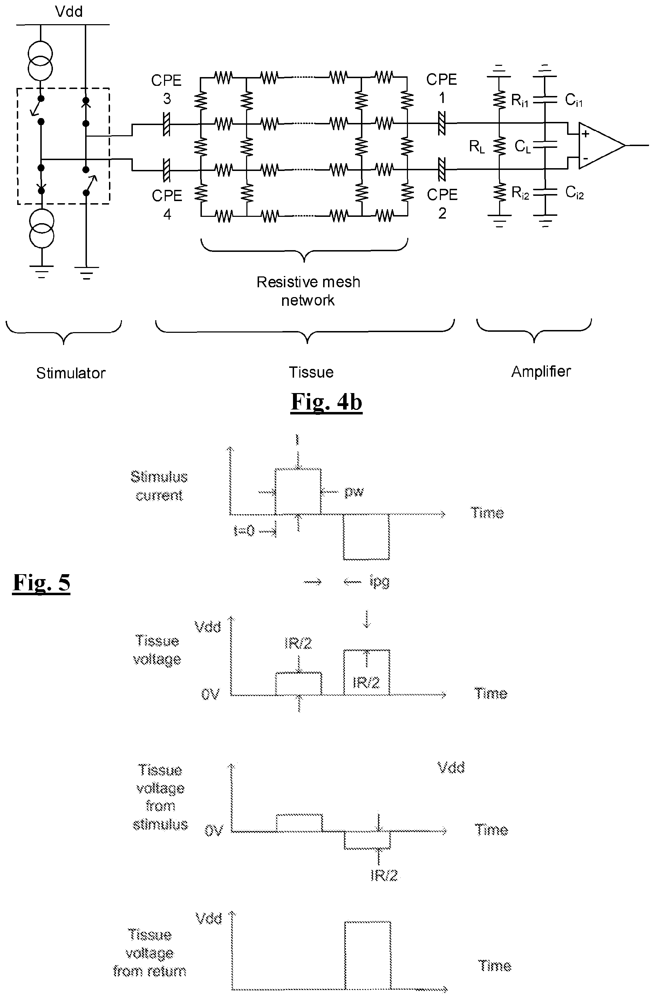

**Fig. 4a** depicts an example configuration of stimulus and recording electrodes for ECAP recording. This configuration utilises an implantable electrode array 450, stimulus electrodes 402, 403, an evoked response measurement electrode 406, a measurement reference electrode 408, and an amplifier 428. The patient's tissues, consisting of an ionic solution, are modelled by a resistor network, shown in **Fig. 4b****,** which depicts an electrical model of the configuration of Fig. 4a. The electrode-tissue interface, which is a metal-electrolyte interface, is modelled by a set of constant-phase elements (CPEs) denoted CPE1, CPE2, CPE3 and CPE4. The model of Fig. 4b contains enough elements to reason about various aspects of the present technology, however the exact choice of model is not critical and additional or alternative modelling elements may be used such as elements of Fig. 3 and/or elements disclosed in the aforementioned WO2020/082126.

Figures 4a and 4b show a system for stimulating tissue and recording an ECAP. The tissue is shown as a resistor mesh to which the electronic components connect via constant-phase elements (CPEs). This representation is explained in detail in the aforementioned WO2020/082126. This consists of: a current driver consisting of two current sources and connections to Vdd and ground, and an amplifier 428 that has finite input impedance with both capacitive and resistive components both directly between the inputs and to ground. The stimulator and amplifier connect via electrode contacts (constant phase elements) to a resistive mesh representing tissue.

In Fig. 4b, the system stimulus electrodes 402, 403 are represented by CPE4 and CPE3 respectively, and the recording electrodes 406, 408 by CPE1 and CPE2. Connections to CPE1, CPE2 etc. will be referred to as electrode 1, electrode 2 etc.

Figure 5 illustrates stimulus waveforms seen on the recording electrodes 406, 408. The first panel of this Figure shows the current through the tissue. The second panel shows the voltage at a point in the tissue distant from the stimulation with respect to the implant ground point. The third panel shows the tissue voltage resulting from the stimulus current. The fourth panel shows the voltage on the implant return electrode (electrode 3 of Fig. 4b) with respect to the implant ground. This is the same as the tissue voltage resulting purely from the connections of the return electrode to Vdd or ground and is independent of the stimulus current. The stimulator in this system can produce stimuli with one or more phases by operation of the switches and current sources. The current amplitudes and switch timing are controlled by the stimulator, and so the current amplitude and switch timing are known prior to stimulation occurring.

The operation of the switches connecting the tissue to Vdd or ground causes currents to flow through CPE1 and CPE2 and into the amplifier resistive and capacitive input impedances. Again, the timing of the switching is known prior to the stimulation occurring.

The method described in this disclosure is relevant to situations when the recording electrodes associated with CPE1 and CPE2 are sufficiently distant from the stimulation sites that that can be considered to be at the same potential, at least on the tissue connecting side. That is to say, the current flow through the resistive mesh close to the recording electrodes is effectively zero although it will include a very small component that is involved in changing the potential of the recording electrodes relative to the amplifier ground point. For example, if the amplifier input capacitance is 20pF, and the voltage changes by 5V, then 100pC of charge must flow. This is small compared to the charge of typically 1uC required to induce an evoked response.

When the system is delivering an anodic stimulus to electrode 4 (the first phase in the waveforms of Fig. 5), it connects electrode 3 to ground, and electrode 4 to the positive current source. The voltage on the recording electrodes 1 and 2 will be somewhere in between the voltages on electrodes 3 and 4, depending on the details of the mesh. For convenience, it will be assumed that this fraction is one half i.e. midway in between. This has been observed by the inventors to be commonly the case. If the impedance between the stimulus electrodes is R, then this voltage will then be IR/2.

When the system is delivering a cathodic stimulus to electrode 4 (the second phase in the waveforms of Fig. 5), it connects electrode 3 to Vdd, and electrode 4 to the negative current source (as shown by the switches in Fig. 4b). Again, the tissue voltage is assumed to be midway between these.

When the tissue is not being driven, the tissue can be connected to Vdd or the implant ground, and in this case, the implant ground will be chosen though it is arbitrary. Choosing ground is the preferred method as this does not require maintaining a high-voltage power supply when not stimulating.

In the case of a biphasic stimulus, the tissue waveform is as per Fig. 5 second panel. It will be observed that this waveform is the sum of the electrode to power supply voltage (fourth panel) and the tissue voltage resulting from the stimulus current (third panel).

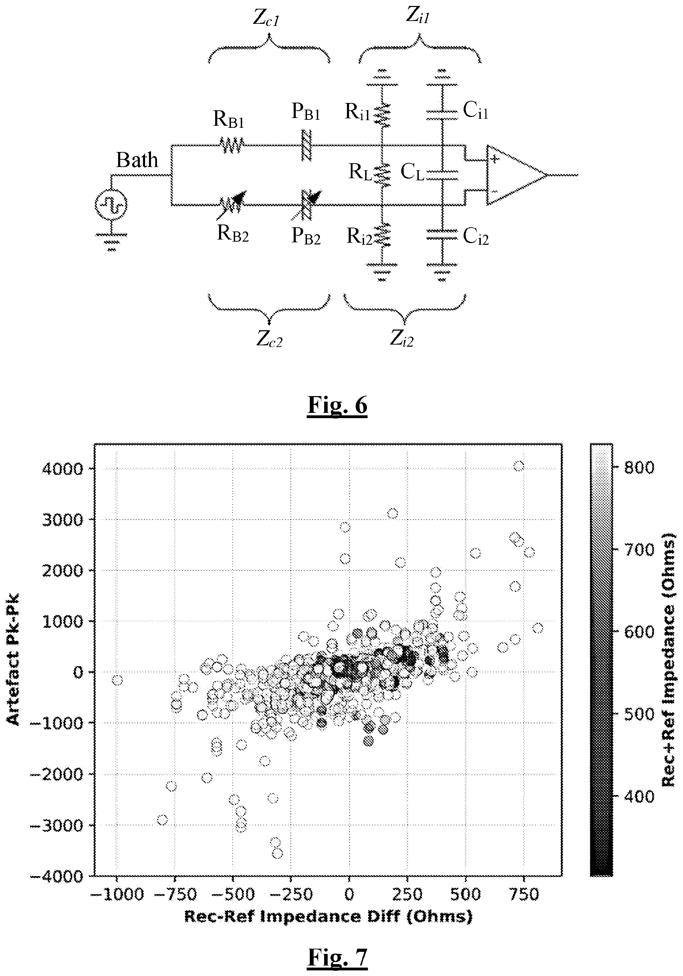

This permits circuit reduction. Since the voltages on electrode 1 and electrode 2 are the same, and the tissue waveform is as described in Fig. 5, then Fig. 4b can be simplified to the simplified circuit of Fig. 6. If we assume that by careful design and component control R_{I1}= R_{I2} and C_{I1}= C_{I2} then there will only be a non-zero voltage at the amplifier output if P_{B1}!= P_{B2}, i.e. a mismatch between the impedances of CPE1 and CPE2. This would be expected in practice, because these electrodes are placed in tissue and it is not homogenous, so it would be expected that these impedances will not be the same. Put another way, for the purpose of analysing artefact, Fig. 4b can be reduced to that in Fig. 6 by noting that artefact has been observed even with no current applied, so to analyse the reason for this the current sources are ignored; they are disconnected during measurement so their CPE can be ignored; for electrodes distant from the stimulating site, the currents are low so all mesh internal nodes are at the same voltage so can be considered as all connected together; the complex mesh of resistances can then be reduced to just two resistors in series with the amplifier inputs representing the source impedance of the mesh as seen through these nodes. These source resistances, labelled R_{B1} and R_{B2} in Fig. 6, may differ in value as represented by the arrow through R_{B2}. In series with each will be a CPE, labelled P_{B1} and P_{B2} in Fig. 6, and these may not be the same value, as illustrated by a second arrow through P_{B2}. Finally, the amplifier has some input impedance to ground which has resistive and capacitive components at each input, and there will be impedance (labelled as R_{L} and C_{L} in Fig. 6) between the inputs causing current to flow that does not flow to ground.

If we now calculate the voltage *V*₁ on amplifier input 1 resulting from a voltage of 1 V at the tissue, calling *Z*_{*i*1} the impedance of the input circuit and *Z*_{*c*1} the impedance of the source resistance and CPE, as illustrated in Fig. 6, so as a simple voltage divider: $\begin{matrix}{V}_{1} = \frac{{Z}_{i 1}}{{Z}_{i 1} + {Z}_{c 1}} \\ = \frac{1}{1 + \frac{{Z}_{c 1}}{{Z}_{i 1}}}\end{matrix}$ Equation 1

Since *Z*_{*i*1} >> *Z*_{*c*1}, using Newton's approximation: (1 + *p*)*^{q}* = 1 *+ pq* if *pq* << 1, we can write: ${V}_{1} ≅ 1 - \frac{{Z}_{c 1}}{{Z}_{i 1}}$ Equation 2

The differential voltage between the amplifier inputs is therefore given by ${V}_{1} - {V}_{2} ≅ \frac{{Z}_{c 1}}{{Z}_{i 1}} - \frac{{Z}_{c 2}}{{Z}_{i 2}}$ Equation 3 Therefore, if the input impedances *Z*_{*i*1} and *Z*_{*i*2} are equal, the differential voltage is proportional to the difference between the CPE impedances. Since Z_{c1} and Z_{c2} are fractional poles, this differential impedance Δ*Z*_{*c*12} will also have the properties of a fractional pole.

Artefact thus appears at amplifier outputs when the impedances of the CPEs differ. This can happen due to uneven tissue around the electrodes. This has been observed to increase over time as scar tissue forms around implanted electrodes, but can also be present at the time of implant. Indeed, the present inventors have observed artefact at near-zero current in human patients, and have also observed that artefact is worse when there is impedance mismatch between the recording electrodes. Fig. 7 is a plot of artefact observed in human patients, relative to absolute electrode impedance and relative to the electrode impedance difference (Z_{c1} - Z_{c2}). It is observed clinically that electrode impedances vary, and that high and mismatched impedances are associated with high artefact. Patients exhibiting a low impedance on the recording electrodes (dark points) tend not to exhibit high artefact. Patients exhibiting a high total impedance on the recording electrodes (light points) may or may not suffer from high artefact. However it can be observed that a larger magnitude difference between recording electrode impedances (points at far left or far right of Fig. 7) tend to exhibit a larger magnitude of artefact.

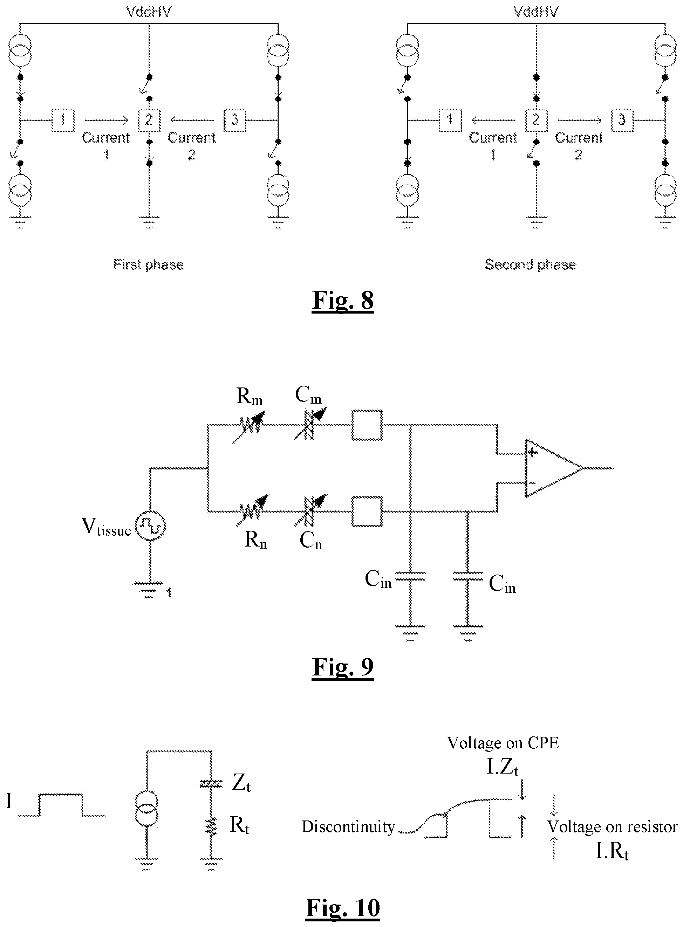

In many neural stimulators an 'H-bridge' is used to generate stimulus pulses, as illustrated in **Fig. 8****.** An H-bridge allows the generation of bi-phasic pulses with currents distributed between multiple electrodes in a scaled manner. For example, in the example in Fig. 8, and assuming these electrodes are three of an 8-contact array numbered 1 to 8, if current 1 and current 2 are in the ratio of approximately 2: 1, then this creates a spatial null in the voltage field to the right of electrode 3 which is advantageous to electrodes 4 and 5. This is discussed further in the present Applicant's International Patent Publication No. WO2020/082126.

However, it will be observed in Fig. 8 that even when no current is flowing, or a low current is flowing, then electrode 2 is switched between VddHV and ground. This induces a voltage between the recording electrodes (via the patient's tissue), and ground. This is illustrated in **Fig. 9****.** If the CPE impedances do not match, which is commonly the case due to uneven tissue growth around individual contacts, then this induces a net voltage at the amplifier, which results in artefact.

The present disclosure recognises that this mismatch can be compensated by several aspects of the present technology, including designing the amplifier for low intrinsic input capacitance, adding additional impedance at the amplifier inputs, using an isolated amplifier module, interposing a driven shield around the amplifier input wires, or treating the electrodes. These are all means to drive the ratio of the input impedance of the amplifier to the difference in impedances of the electrode inputs above a predetermined level (see Equation 3) and thereby compensate for the mismatch in the CPE components of the electrode impedances. A key insight presented by the present disclosure is to present a framework by which to predetermine the level. To this end, we first describe methods to determine the desirable input impedance of the amplifier. This requires novel circuit theory developed specifically for this purpose, which is explained prior to the calculation.

A CPE has an impedance that can be written in the Laplace domain as: ${Z}_{CPE} = \frac{1}{{C}_{F} {s}^{α}}$ Equation 4 where *C_{F}* is its impedance at a frequency *ω* = 1 *rad*/*sec* and 0 < *α* < 1, and typically *α*~0.5.

Taking the inverse Laplace transform shows that the step response of a CPE to a current step of magnitude *I*₀ is: ${V}_{s} \left(t\right) = \frac{{I}_{0}}{{C}_{F} Γ \left(α\right)} \frac{{t}^{α}}{α}$ Equation 5 This can be demonstrated experimentally with a saline bath, some platinum iridium electrodes and standard laboratory equipment, although the term *αC_{F}*Γ(*α*) will appear as a constant.

The response of the CPE to a current impulse with charge Q, is: ${V}_{I} \left(t\right) = \frac{Q}{{C}_{F} Γ \left(α\right)} {t}^{α - 1}$ Equation 6 Note that the impulse response is the derivative of the step response (as expected as a common mathematical identity), though it is not defined at t = 0. By combining these equations: For $\begin{matrix}{V}_{I} \left(t\right) = {V}_{S} \left(t\right) \frac{Qα}{{I}_{0} t} \\ t \neq 0\end{matrix}$ Equation 7

This equation is deceptively simple. It shows how we can easily calculate the expected response to a rectangular voltage pulse driven through a capacitor given measured behaviour when driven with a rectangular current pulse driven through a resistor. Since the latter is much simpler to measure due to the lower dynamic range of measurement required, this is very convenient.

If a sharp edge, such as occurs at the beginning and the end of a rectangular voltage pulse, were to be driven through a capacitor to a CPE, it will generate an impulse of charge and a response *V_{I}*(*t*) according to Equation 6, with sign depending on the sign of the edge. Such capacitive impulses occur when the H-bridge of Figure 8 switches, even when no stimulation is present and the current sources are inactive. It has been observed that artefact in human patients can exceed 1 mV in this situation. The capacitor providing the pulse is formed from the input impedance of the amplifier. A negative impulse of charge, such as results from the falling edge at the end of a voltage pulse driven through a capacitor, will cause a spike of negative voltage across the CPE which decays to zero over time, and a complementary decaying positive spike of voltage across the input impedance of the amplifier. It is this spike that appears as artefact. Estimating the voltage (*V_{I}*(*t*)in Equation 6 above) across the CPE resulting from an impulse of charge, which may be done using Equation 7, is therefore tantamount to estimating the amount of artefact at the amplifier input.

As an example of use of Equation 7, consider the case where the step response measurement of a CPE with ∝= 0.7 showed a voltage of *V_{S}*(*t*) = 18*mV* after 200*us* driven with a current of *I*₀ = 1*mA* for *t* = 200*us.* If the same CPE were then driven with a 15V step through a 100pF capacitor i.e. a charge *Q* = 15*V* × 100*pF*, then the voltage at 200us will be ${V}_{I} \left(t\right) = 18 mV \frac{\left(15V\times 100pF\right) \times 0.7}{\left(1mA\times 200us\right)} = 94.5 uV$ Equation 8

This represents a practical way that predictions can be made about the voltages appearing on a CPE in response to a charge impulse. Since such voltages are the basis of artefact, this is very useful.

One aspect of the present technology is a method of determining a design criterion for measurement circuitry including the measurement amplifier, in order to constrain artefact below a desired level for a given impedance mismatch scenario.

Fig. 9 illustrates the essence of this problem, by showing the simple case of an amplifier with input capacitance C_{IN} on each input. In this case the impedances Zi₁ and Zi₂ have been assumed to be capacitive, and equivalent to C_{IN}. This simplification is to aid explanation only.

The tissue voltage is V_{TISSUE}. The tissue and electrode impedances have a resistive part (Rₘ and Rₙ) and a fractional pole part (Cₘ and Cₙ). The resistive parts Rₘ and Rₙ are, for SCS, most commonly around 350 ohms each. In this circuit, current through them is dominated by C_{IN} which will be ~100pF or less, with the desired value to be determined. When the stimulus ceases, current does not flow through the resistive components so they do not generate appreciable voltage. However, after stimulation has ceased, the voltage changes across the CPE elements Cₘ and Cₙ generate artefact as charge redistributes within them. The voltage across these CPEs is set by how much current flowed through them during the stimulation, which is controlled by C_{IN} and by the driving voltage VddHV.

We now further consider the electrode properties. After implantation, tissue grows around electrodes and changes their impedance. This can be modelled in a laboratory by painting electrodes with random patterns using a non-conductive paint such as nail varnish. Electrodes can then be placed in a saline bath and the resistive and CPE parts of their impedance can be measured.

The resistive and CPE parts of an electrode's impedance can then be measured as shown in Figure 10. A current step is applied to the electrode using a large return electrode, so the impedance measured is only from the electrode under test. The response shows a step at the start and thereafter a further rise as current is accumulated on the CPE. The voltage on the CPE must not exceed 400mV because above this voltage non-linear effects such as electrolysis occur. The resistive and CPE components can be easily identified using an oscilloscope.

Knowing the current, the impedance of each component can be calculated and expressed in ohms. The CPE impedance (voltage / current) changes with pulse width but this does not create complications as long as the same pulse width is used for all measurements.

To determine the required input impedance of the amplifier to obtain the desired artefact, it is necessary to know the electrode impedance components (resistive and CPE) of implanted electrodes. These can be measured clinically and values in the range 250 ohms to 900 ohms are observed for SCS. These are measured from peak voltages, and do not give insight into the relative contribution of the resistive and CPE components, and these must be determined.

Since the electrode is cylindrical, there is not a closed form solution of the impedance between it and surrounding saline (or tissue). However, key insights can be obtained by considering a spherical electrode as the impedance for a sphere can be calculated and provides guidance on the behaviour to be expected.

The resistive component of impedance is generated by the bulk resistivity of the saline. So it is possible to calculate the impedance of a sphere in a homogenous bath, measured against a large reference electrode at infinity, as: ${Z}_{R} = ρ {\int }_{r = R}^{r = ∞} \frac{dr}{4 {πr}^{2}} = \frac{ρ}{4 πR}$ Equation 9

This indicates that the resistive part of impedance varies with the reciprocal of the radius R.

As an alternate method of measurement, two identical electrodes in series can be measured and the values halved.

The artefact caused by current into the amplifier input impedance is generated by the constant phase element portion of the electrode impedance which covers the electrode surface. This impedance is inversely proportional to the surface area of the electrode, which goes to the square of its radius. i.e. as an electrode gets smaller the reactive (CPE) part of its impedance increases faster than the resistive part. This is shown in Equation 10, where k is the conductance per unit area. ${Z}_{CPE} = \frac{1}{k 4 {πR}^{2}}$ Equation 10

This indicates that the change in impedance of the CPE goes to the square of the change in impedance of the resistive component. In cases where, as is commonly seen, an electrode impedance changes by 3x, the CPE impedances changes by 9x. The change in CPE impedance is not apparent when only simple methods are used to measure impedance, such as measuring the peak-to-peak voltage for a square wave and dividing by current.

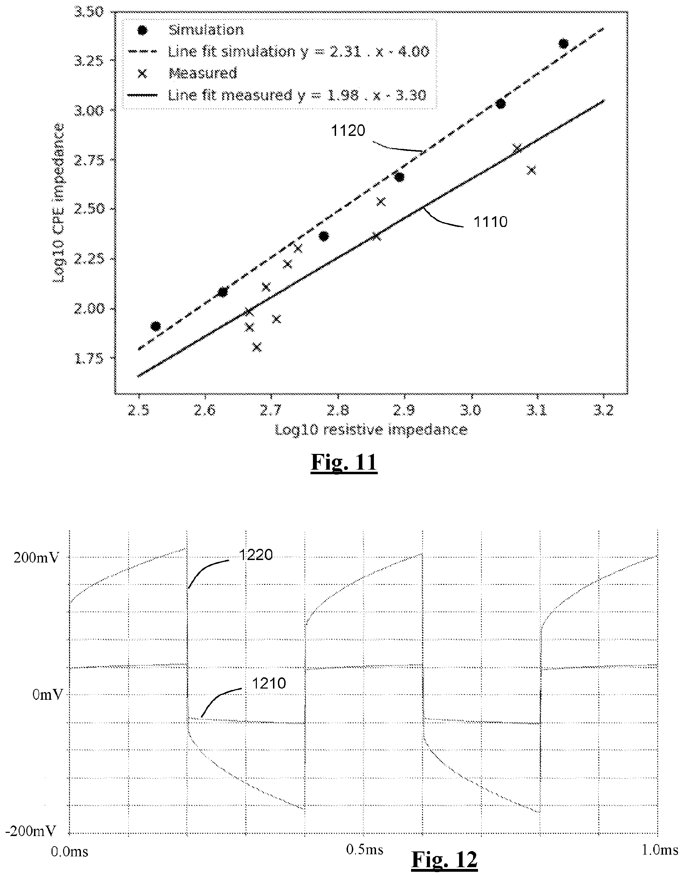

To test if this geometric model applies to actual electrodes, standard SCS electrodes were painted in random patterns with nail varnish and their impedance components were measured. In a second experiment, electrodes were simulated using the method of Scott and Single (Jonathan Scott and Peter Single, "Compact Nonlinear Model of an Implantable Electrode Array for Spinal Cord Stimulation (SCS)", IEEE Transactions on Biomedical Circuits and Systems, vol.8, no.3, pp.382-390, 2014) and these components measured. The results were then plotted on log-log scale (Fig. 11) and the underlying relationship between the resistive and CPE parts were measured by fitting line 1110 to the measured data and line 1120 to the simulated data. The slope of this line (approximately 2 for both simulated and measured impedances) supports the use of Equations 9 and 10.

This relative change in the resistive and CPE parts of the impedance for a 3mm and 200uM electrode are illustrated in Fig. 12; note how the proportion of the CPE and resistive components varies with electrode size. Where the resistive component changes by about 3X between the 200um electrode trace 1210 and the 3mm electrode trace 1220, the CPE component changes by a much greater degree. From Fig. 11 it can be inferred to be about 9X.

It was necessary to conduct these measurements with saline baths and simulators as there is limited access to human subject implanted electrodes. The clinical systems available do not separate the resistive and CPE parts of impedance. Figure 12 shows pulse waveforms of electrodes of differing physical length, and this illustrates the difficulty of measuring the CPE component - for the 3mm electrode it is a very small fraction of the total impedance.

Having demonstrated that the resistive part of an electrode impedance varies with 1/*R* and the capacitive (CPE) part by 1/*R*², a model of electrode impedance can be constructed as follows: if we denote *r* as the normalized resistive part of the impedance, and *c* as the normalized CPE part, then the total impedance of two electrodes with (unknown) scale factors m and *n*, for some *r* and *c* will be: ${Z}_{n} = rn + {cn}^{2}$ Equation 11 ${Z}_{m} = rm + {cm}^{2}$ Equation 12

Relating this to Fig. 9: ${R}_{m} = rm$ Equation 13 ${C}_{m} = {cm}^{2}$ Equation 14 With similar equations existing for *n*.

The two electrodes have a difference in impedance *Z_{d}* ${Z}_{d} = r \left(n-m\right) + c \left({n}^{2}-{m}^{2}\right)$ Equation 15
and a total impedance *Zₜ*: ${Z}_{t} = r \left(n+m\right) + c \left({n}^{2}+{m}^{2}\right)$ Equation 16

The difference in their CPE impedances, which leads to artefact is $Δ {Z}_{C 12} = c \left({n}^{2}-{m}^{2}\right)$ Equation 17

Since the total impedance of an electrode can be as determined clinically to be 200 to 800 ohms, the impedance between two electrodes in series is in the range 400 to 1600 ohms. But artefact is worst when the impedances don't match, so the range of 400 to 1000 ohms need only be considered. This includes impedances such as 200 & 800, 400 & 400 and 800 & 200 and so on.

The CPE impedances can be measured in a saline bath. With no varnish, the CPE impedance, *Z_{CPE}* of a 3mm long x 1.3mm diameter platinum iridium electrode as commonly used for SCS was found to be 18 ohms, or 18mV change in voltage for a 1mA 200us pulse. (Note the similarity to the 20 ohm value used in the prior art.)

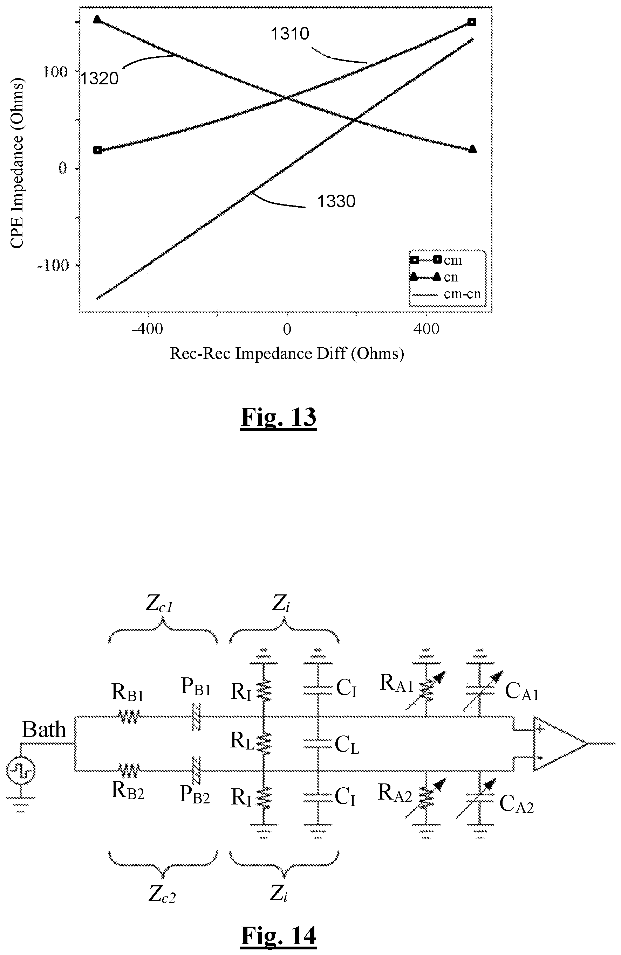

Solving Equation 15 through Equation 17, and using 18 ohms as the nominal CPE impedance *c* and plotting the resulting values of *Cₘ*, *Cₙ*, and Δ*Z*_{*C*12} *= Cₘ - Cₙ* provides the traces 1310 (*Cₘ*), 1320 (*Cₙ*), and 1330 (*Cₘ - Cₙ*) in the graph in Figure 13. The relevant observation from this is that the impedance of the CPE increases from the nominal 18 ohms to 150 ohms, a factor of *A* = 8.3. This increase by a factor of *A* in the CPE impedance component of an electrode due to tissue growth means the step response *V_{S}*(*t*) of an electrode with tissue growth will be greater by a factor of *A* than a measurement of the step response made on the same electrode without tissue growth.

Incorporating this factor A into Equation 7, when measurements of *V_{S}*(*t*) are made with an electrode without tissue growth, and putting *Q = V_{ddHV}* × *C_{IN},* the predicted artefact is: ${V}_{I} \left(t\right) = {V}_{S} \left(t\right) \frac{Qα}{{I}_{0} t} A$ Equation 18

This data was taken with an amplifier an input capacitance of 100pF.

Evaluating Equation 18, having observed an 18mV rise with 1mA over 200us (a CPE impedance of 18 ohms), the peak artefact 200us after the stimulus, where the artefact is induced by a 15V pulse (the swing of VddHV) with 100pF of stray capacitance per input will be: ${V}_{s} = \frac{18 mV \times 8.3 \times 15 V \times 100 pF \times 0.7}{1 mA \times 200 us} = 0.78 mV$

In this equation, the 15V represents the switching of the tissue voltage compared to the implant ground as a result of the H-bridge. The 0.7 is the pole fraction number α for the electrode. This is comparable to the value measured for this electrode in human subjects with similar impedance mismatch.

To then achieve an artefact less than 100uV, requires that the input capacitance be less than a limit given by: $C = \frac{100 uV}{780 uV} \times 100 pF = 12.8 pF$

Of course, the acceptable value of 100uV for artefact is arbitrary and in other embodiments more or less may be tolerable depending on circumstances.

This completes the description of determining the design criterion for the measurement amplifier according to one aspect of the present technology. It will now further be demonstrated that multiple aspects of the present technology can be used to achieve the acceptable value for artefact if the intrinsic input capacitance does not do so on its own.

One aspect of the present technology is based on the observation that the voltages at the amplifier inputs would be equal, and artefact would be zero, if the terms of Equation 3 were equal. If the input impedances Zᵢ₁ and Zᵢ₂ were to be made separately adjustable, for example under software control, then it would be possible to choose them so that Equation 20 is true (or approximately true) and artefact would be reduced to zero, or close to zero, thereby compensating for mismatch in the electrode CPE impedances. $\frac{{Z}_{c 1}}{{Z}_{i 1}} = \frac{{Z}_{c 2}}{{Z}_{i 2}}$

This can be achieved by placing adjustable components in parallel with Zᵢ₁ and Zᵢ₂. These can contain both resistive and capacitive elements (*R_{Ai}* and *C_{Ai}*) which can be independently adjusted to match the division ratio, as illustrated in Figure 14.

Adjustment could be performed in a clinic by a clinician, by measuring the artefact at a current below the patient's threshold, and adjusting the adjustable component impedances until this is nulled. This could also be done automatically by the implant, during periods when the patient is not using their device, or even between stimuli for example by way of a feedback loop.

There are several ways by which the adjustable components *R_{Ai}* and *C_{Ai}* of Fig. 14 could be added. If the amplifier for example has an input capacitance of approximately 50pF, then providing an adjustable capacitance of up to 500pF would be desirable. This could be achieved with components mounted on a PCB, or incorporated in an ASIC, with the latter approach being preferable. A switched array with values taking a binary series would be preferable e.g. 25pF, 50pF, 100pF, 200pF, 400pF totalling 475pF when all are connected in parallel.

Where the implant for example has an input resistance of several gigohms, suitable resistors could be fabricated in an ASIC using transistors that are turned on weakly. Or if the artefact contribution of the capacitive component dominated the resistive component, then additional resistance may not be necessary. However, it is considered here for completeness.

A third aspect of the present technology for compensating for mismatch of CPE impedances is based on the observation that the amplifier input impedance consists of the following components: (i) the impedance of the transistors etc. from which the amplifier is composed. This can have both a capacitive and resistive component, where the resistive component will usually provide amplifier bias. (ii) Stray capacitance from the electrical pathway from the electrode contact to the amplifier inputs. This latter capacitance consists of capacitance from one electrode wire to the adjacent wire, and from electrode wires to the point designated 'earth'. (iii) Other circuitry that might be added such as capacitance between electrodes to limit current during MRI procedures, capacitance between the lead wires in an epidural lead etc.

The capacitance between contact connections does not induce artefact, as all the wires are connected to tissue, so they move in unison so there is no net voltage between the wires so the current flow contribution though the CPEs is zero. However, the second capacitance, as outlined above, does cause current to flow through the CPE which then causes artefact.

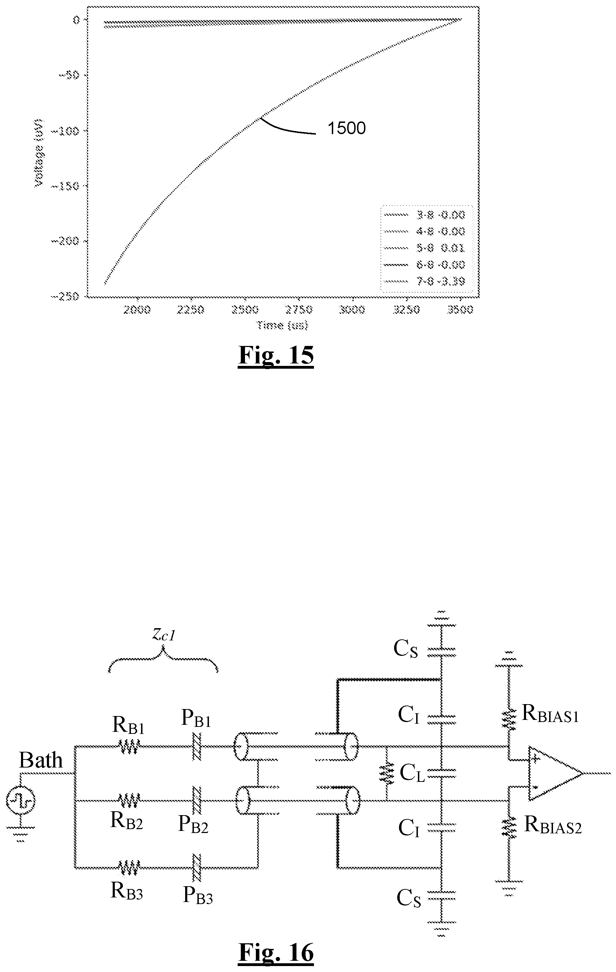

This is demonstrated in the plot of Fig. 15 which shows the artefact recorded on leads in which all contacts have 3mm length except for electrode 7 which is 0.5mm. The trace 1500 obtained from electrode 7 is intended to show the effect of the high impedance electrode. In this case every contact has 100pF to ground.

The third aspect comprises creating a shield around the conductors conveying the electrode potentials to the amplifiers, and driving this shield with a voltage that is equal to that of one of the unused electrode contacts. This then decreases the apparent stray capacitance to the amplifier input by means of the Miller effect.

Fig. 16 illustrates one implementation of the third aspect of the present technology. In this case one of the electrodes (represented by R_{B3} and P_{B3}) is allocated to be the shield connection. This electrode is connected to a shield placed under and around the electrode wires to the point that they reach amplifier inputs. This shield can consist of flexible cable shield, of PCB planes placed under and over PCB tracks, or of metal planes in an ASIC placed under and over the ASIC wires. This will provide the desired effect of driving the shield to follow the bulk tissue voltage. While this is at a cost that one electrode cannot be used for recording, many implants have surplus electrodes. This also fails to provide bias current for the amplifiers in cases where this is needed. In some cases the amplifier input impedance is entirely capacitive so this is not a problem, but in other cases this resistance will create artefact.

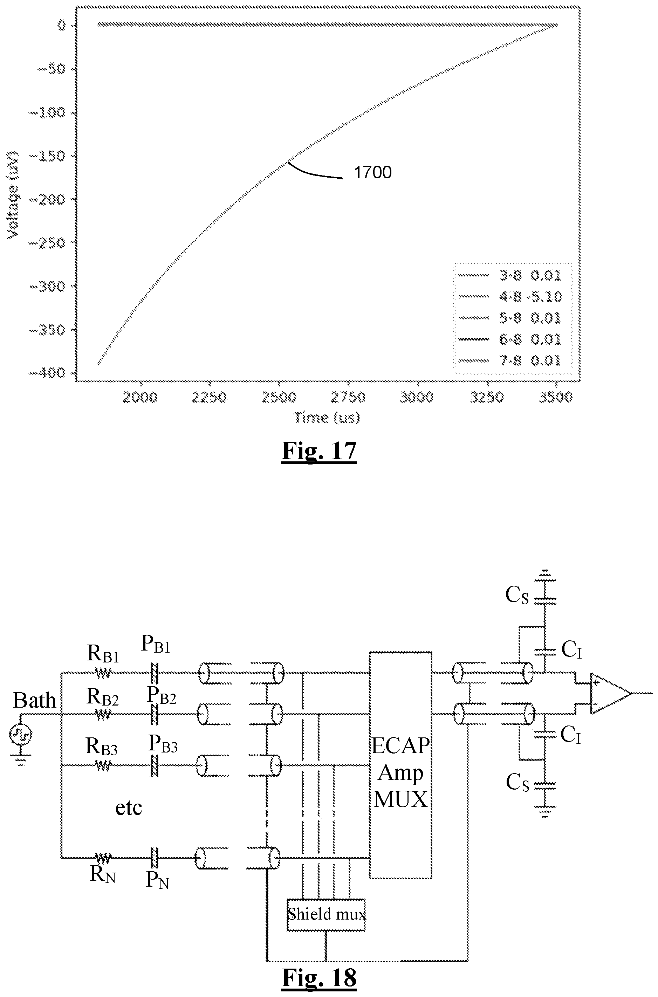

The capacitance from the shield to ground is estimated to be equal to the previous capacitance to ground. As shown in Figure 17_, this works for the recording electrodes with the artefact on the electrode driving the shield to be degraded. This shows a simulation of the situation when the shield is connected to Electrode 4. Artefact on electrode 7 is reduced by a factor of 10 compared to the previous situation shown in Figure 15, whereas artefact on electrode 4 (represented by the trace 1700) has increased, as expected since that electrode is driving the shield. However, this increase is inconsequential as electrode 4 is not being used for measurement.

Figure 18 shows an implementation of the third aspect where a multiplexor (labelled Shield mux) selects the unused channel to drive the shield. A second multiplexor (labelled ECAP Amp MUX) selects which channels to use for ECAP recording.

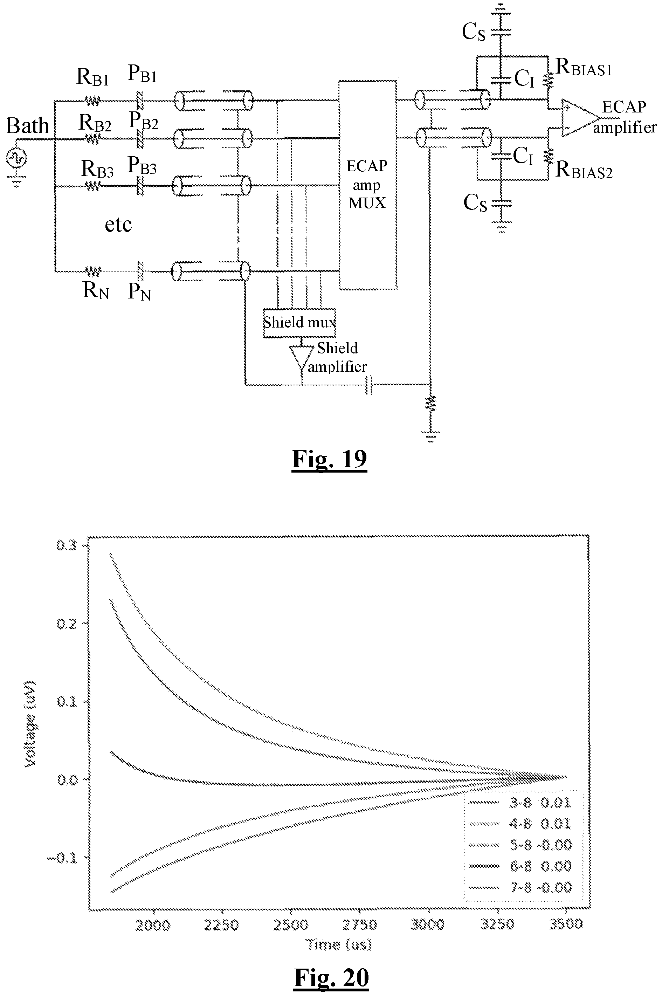

Figure 19 shows the case where a multiplexor (labelled Shield mux) is used to select the electrode to be used as the shield reference and this is buffered before use by an amplifier labelled Shield amplifier. In addition, a high-pass filter and resistor provide a DC path to ground that provides bias to the ECAP amplifier. The buffer amplifier does not have to have high performance characteristics e.g. low noise, as it is only driving the shield. Any noise from this amplifier becomes a common-mode signal at the amplifier input, after being coupled in through the divider consisting of the shield capacitance per channel and the tissue output impedance. A high-pass filter separates the bias of the ECAP amplifiers from that of the shield amplifier.

This is very effective, reducing artefact to less than 1uV pp as shown in Figure 20.

The shield amplifier does not have to have exceptional performance characteristics, as its noise becomes common mode noise for the ECAP amplifier. The resistance of the bias resistors of the ECAP amplifier are amplified by the Miller effect. This has a great benefit in widening the range of amplifiers that are suitable, as is discussed in the following.

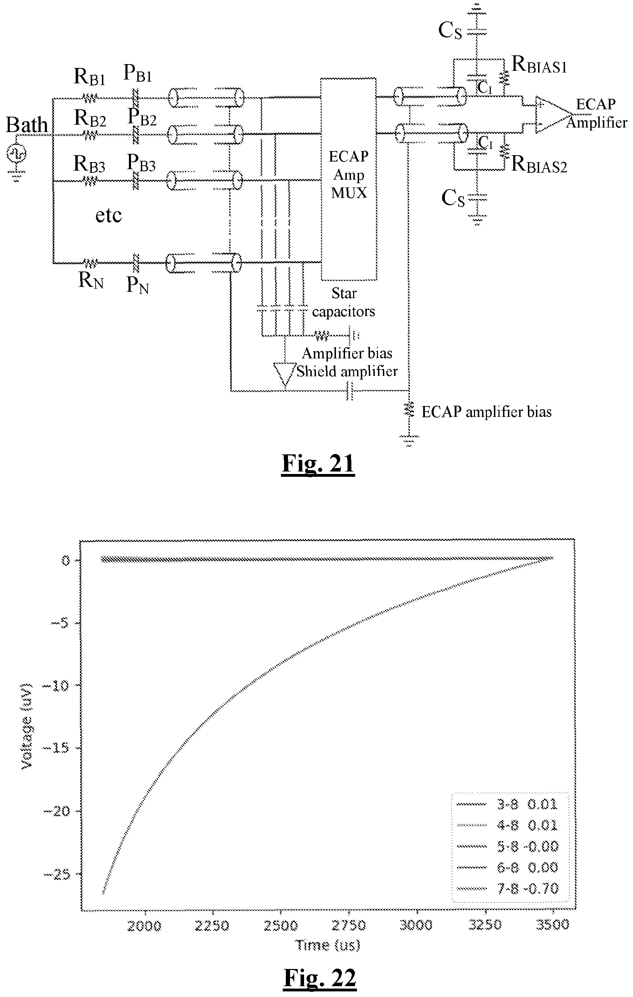

Another implementation of the third aspect may comprise a SCS stimulator having a star-connected capacitor array between the electrodes in place of the shield multiplexor. A typical value for such capacitors is 100pF. At the common point of connection of these capacitors, for a system with 12 channels, this will behave like a voltage source whose output impedance is the parallel connection of a single electrode impedance (typically 500 ohms) and 100pF per channel 40 ohms of resistive impedance and 1.2nF of capacitance. This star point can then be used to drive the shield. This would be suitable for use in an implant, where the stray capacitance might be approximately 100pF per channel. Figure 21 shows the circuitry required to perform this function. Figure 22 shows a simulation of this and shows that the circuit performance is adequate. (The artefact is large on electrode 7 because that electode is simulated as narrower then the others.) This is implementation does not degrade the performance of any channel, rather it spreads the load across all of them.

In simulating this circuit, star capacitors of 100pF, and a shield amplifier bias resistor of 10 megohms has been found to be preferable. In simulation, this induces 25uV pp of artefact on a 3mm electrode. Given that a typical ECAP amplifier has a dynamic range exceeding 2mV, this is an acceptable result.

A further issue to consider is amplifier choice. In choosing an amplifier for measuring evoked responses there is normally a trade-off between noise and bandwidth at low frequencies. All amplifiers require bias current, and the bias current flowing through the input bias resistors produces an offset. For example, consider an amplifier with a target input impedance of 1 megohm. Using an AD4625 as the amplifier, an input offset of 50uV is obtained. The LT6018 is a preferable amplifier as it is quieter by around 9dB, but its bias current is prohibitive as can be seen from the following table.

| Amplifier | Noise specification (nV/rt Hz) | Bias offset current | Offset in amplifier of gain 1000 |
|---|---|---|---|
| AD4625 | 3.3 | 50pA | 50mV |
| LT6018 | 1.2 | 50nA | 50V |

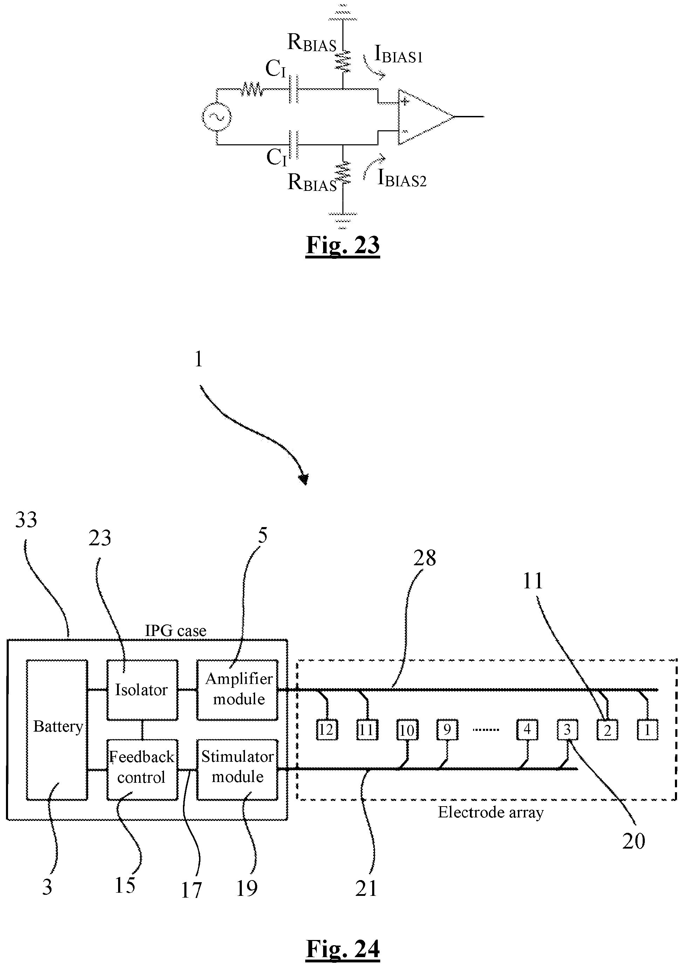

However, when the bias point is driven by an amplifier and follows the tissue impedance, much lower valued bias resistors can be used. As a result, lower bias value resistors can be used as shown in **Fig. 23****,** and the lower noise amplifier (LT6018) can be chosen.

Returning to Fig. 6, it is noted that this model considers the case when the tissue impedance is mismatched, and the tissue is being driven between VddHV and ground, which is normal behaviour at zero current as described in the preceding. Note that the voltage dividers formed, first by P_{B1} and R_{B1} with R_{I1} and C_{I1}, and second by P_{B2} and R_{B2} with R_{I2} and C_{I2}, differ. The mismatched impedance creates a signal at the amplifier output. Due to the large difference between the driving signal (typically 15V) and the ECAP being recorded (typically 30uV but might be as low as 10uV) these differences between impedances are of great significance.

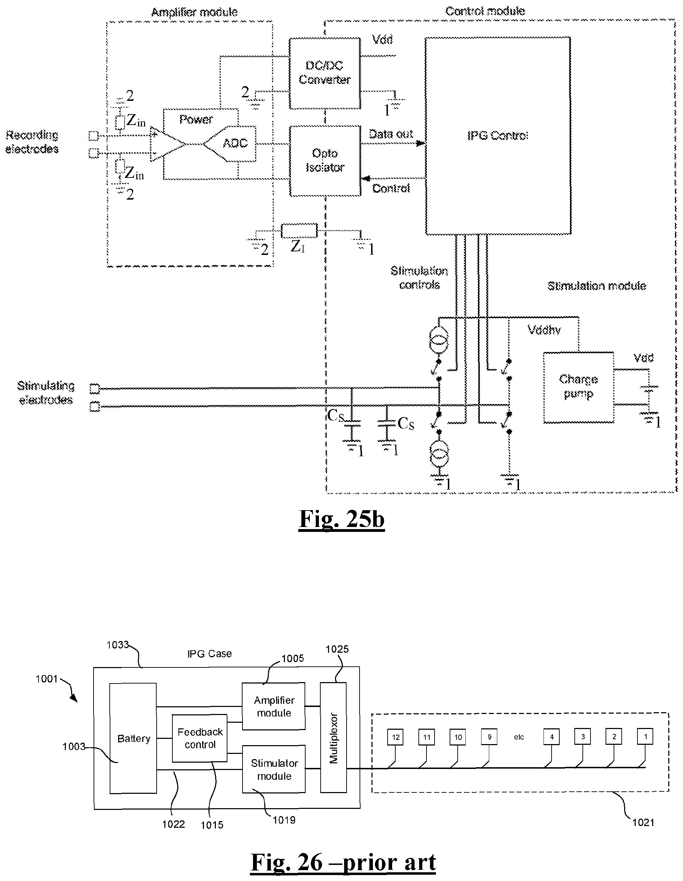

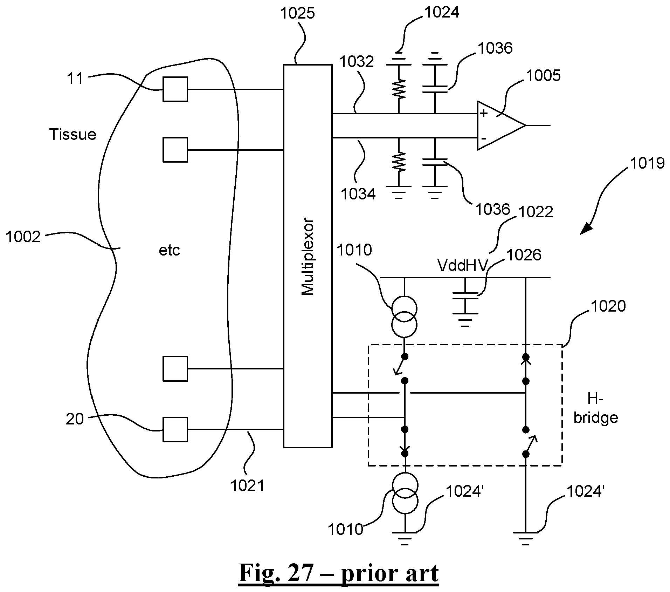

A further aspect of the present technology provides for amplifier isolation. To this end we provide an overview of an existing IPG system. Figs. 26 and 27 illustrate an existing IPG system. The IPG system 1001 includes a power source 1003 in the form of a battery, a feedback control module 1015, an amplifier module 1005, a stimulator module 1019, a multiplexer 1025, and an electrode array 1021.

The power source 1003 provides power to each of the amplifier module 1005, control module 1015 and the stimulator module 1019, whereby they share a common electrical ground. This can include sharing an electrical ground 1024 with a case 1033 of the IPG.

Fig. 27 illustrates a simplified schematic of a feedback controlled spinal cord stimulation (SCS) system. The stimulator module 1019 (in a simplified form) includes two current sources 1010 and a H-bridge switch 1020. The stimulator module connects (with the H-bridge) the tissue 1002 to either the stimulating voltage VddHV 1022, or to ground 1024. The power source 1003 (directly or indirectly) provides power supplies 1022 such as VddHV to maintain a DC potential against ground 1024 that are connected for alternating-current purposes, with capacitors 1026 filtering noise. The ground 1024' for the stimulator module 1019 is conventionally the same as the ground 1024 for the amplifier module 1005.

The amplifier inputs 1032, 1034 include capacitance 1036 to ground 1024. This would normally be "stray" capacitance. In some cases, adding a ground plane underneath sensitive wiring is preferred as this decreases noise coupling from nearby electrical nodes. This adds to this capacitance. Thus the current flowing into the capacitances at the amplifier input 1032 can flow through the stimulus electrode ground connection 1024'.

As described separately herein, the current flowing into the amplifier input impedance through differential tissue impedances creates a differential voltage at the amplifier input. This current stimulates the constant phase elements leading to a residual voltage that appears as artefacts. Using auto-zero mosfet amplifiers, the input impedance of the amplifier 1005 can be made to be almost completely capacitive, leaving the capacitance as the primary path for input currents. This current must go somewhere and the ground 1024 symbol in Fig. 27 illustrates a common point in the circuit, a point that is conventionally shared with other components such as the stimulator module 1019.

### Approaches

One approach to mitigate the flow of amplifier input current to the stimulator module ground 1024' is to design an amplifier with very high intrinsic input impedance (low intrinsic input capacitance) to ground as described above. A second approach is to isolate the amplifier 1005 from the stimulating circuitry/stimulator module 1019. That is, to separate the ground connection 1024 at the amplifier inputs from the ground connection 1024' used by the stimulator module 1019.

### Overview of an example implantable pulse generator with isolator

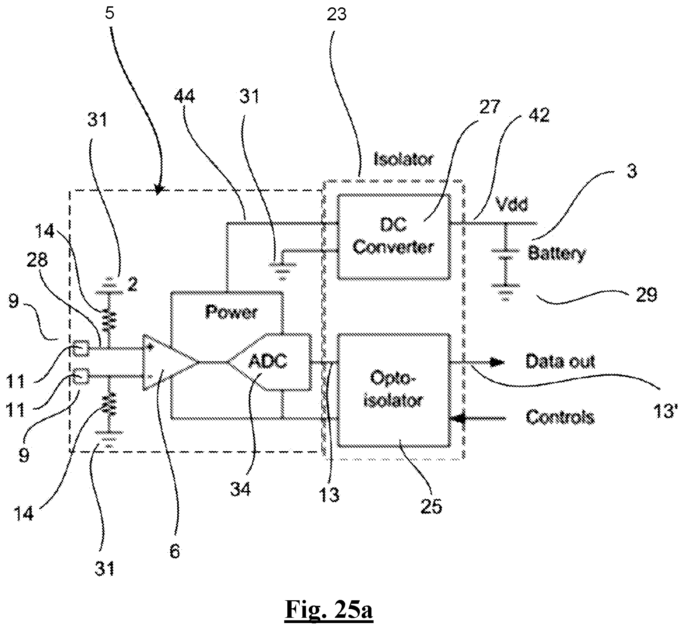

An example of an implantable pulse generator (IPG) system according to a third aspect of the present technology is illustrated in Figs. 24 and 25a and 25b. The IPG system 1 includes an IPG case 33 and one or more leads 28, 21 with electrodes 11, 20. The IPG case 33 contains electronics for stimulation, that can include a stimulator module 19, amplifier module 5, control module 15, a power source 3, and isolator 23. The one or more leads can include a stimulation lead 21 to deliver stimulation current at corresponding stimulus electrodes 20 to the tissue. Measurement electrodes 11 are provided to receive evoked compound action potential (ECAP) 9.

The stimulator module 19, similar in function to the stimulator module 1019, delivers stimulation current to the stimulation leads 21 to stimulate specified areas of a patient's tissue. The stimulation module 19 receives power from the power source 3 to form the stimulation current. The amplitude, type, timing, shape and other characteristics of the stimulation current are based on the control signal received from the control module 15.

The stimulation current evokes an ECAP 9 in the tissue that is received at the one or more measurement electrodes 11 and sent to the connected amplifier module 5. In turn, the amplifier module 5 amplifies the received ECAP 9 (or components thereof) to produce a feedback signal 13.

The feedback signal 13 is received at the control module 15 to provide closed-loop feedback control such that the control module 15 sends a control signal 17 to the stimulator module 19 based on the feedback signal 13. That is, closed-loop feedback on the stimulation current to be produced by the stimulator module 19 is based on the ECAP 9 produced by such stimulation currents.

Although the amplifier module 5 in this example is configured to be inside the IPG case 33, it is electrically isolated from the rest of the electronics 19, 15 by the isolator 23 as will be discussed below. This is best represented in Fig. 25a, that shows the amplifier module 5 that, with isolator 23, is isolated from the power source 3. Furthermore the feedback signal 13, from amplifier module 5, is isolated from the control module 15.

By isolating the amplifier module 5 from the other components, a high isolation impedance (represented as impedance Zi in Fig. 25b) between the amplifier module 5 and the electrical ground 29 (of the IPG case 33 and other components) is achieved to reduce artefact and thereby enhance ECAP detection.

Furthermore, the isolator 23 may be selectively configured with a low coupling capacitance between the amplifier module 5 (providing feedback signal 13) and the control module 15 (receiving the feedback signal 13' passed through the isolator 23). This improves the responsiveness and quality of the feedback signal 13, 13', as it increases the impedance between the amplifier inputs and the amplifier ground, and so decreases the current that flows into the recording electrode wires. This decreases the current that flows through the mismatched constant-phase elements at the electrode tissue interface and thereby decreases the artefact that is recorded by the amplifier.

The components of an example of the system of Figure 24 will now be described in detail.

### Stimulation leads 21 and measurement/reference leads 11

The stimulation leads 21 include electrically insulated leads to deliver stimulation current from contacts (that are electrically connected to the stimulator module 19) to electrodes 20 located at the tissue 1002. The electrodes 20 are surgically, and selectively, located at the tissue 1002 such that stimulation current provides pain relief.

The measurement/reference electrodes 11 may similarly be part of electrically insulated leads 28, but instead the electrically insulated leads deliver ECAPs from the tissue to the amplifier module 5.

Although the example illustrated in Fig. 24 shows a single stimulation lead 21 and a single measurement/reference lead 28, each with multiple electrodes, it is to be appreciated that other configurations can be used. For example, there may be multiple stimulation leads 21. Similarly, there may be multiple measurement/reference leads 28.

In yet another example, a common lead can function as both a stimulation lead and a reference lead. This may be achieved by having multiple wires within the lead with respective electrodes. In some examples, this can include electrodes that are dedicated for stimulation current and others for detecting ECAP.

### The amplifier module 5

Referring to Fig. 25a, the amplifier module 5 includes an amplifier 6 that receives one or more ECAPs 9 from the measurement electrodes 11. The amplifier 6 amplifies the ECAP that is then passed to an analog to digital convertor (ADC) 34 to convert the amplified ECAP to a digital feedback signal 13. This digital feedback signal 13 is then passed to an opto-isolator 25 of the isolator 23.

The amplifier 6 is powered by the power source 3 via the isolator 23, that may include a DC to DC convertor 27 (of the isolator 23) to provide the suitable power for the amplifier 6.

Notably, the first electrical ground 29 of the power source 3 (which is the same common electrical ground with the IPG case 33, control module 15 and stimulator module 19) is different to the second electrical ground 31 at the amplifier module 5.

### The isolator 23

Referring to Fig. 25a, the isolator 23 includes two isolating functions. Firstly, to provide isolating power to the amplifier module 5. Secondly, to electrically isolate the feedback signal 13 (or other control or communication signals to and from the amplifier module 5).

In one example, isolating power is achieved with a DC to DC convertor 27. As noted above the voltage input 42 to the DC to DC convertor 27 is power source 3 that has a respective first electrical ground 29. The power output 44 from the DC to DC convertor 27 to the amplifier module 5 has a respective second electrical ground 31, whereby the second electrical ground 31 for the amplifier module 5 is independent of the first electrical ground 29.

In some examples, a suitable isolating DC to DC convertor includes NKA0303SC optically isolated power supply manufactured by MURATA. This DC to DC convertor has an isolation capacitance of around 20pF. It is to be appreciated that a lower isolation capacitance could be selected based on the specified design of the isolator 23 and system 1.

In one example, isolating the feedback signal 13 is achieved with an opto-isolator 25. An opto-isolator (also known as a photo coupler) typically functions by converting an electrical signal (e.g. feedback signal 13) to an optical signal (for example as using a light emitting diode) and whereby the optical signal is received and converted to an electrical signal (such as using a photodiode). Since the signal passes, at least in part, as transmission of light, this allows the device to electrically isolate between an input signal and an output signal of the opto-isolator 25.

In some examples, a suitable opto-isolator 25 for the feedback signal 13 includes MID400 isolated driver manufactured by ON SEMICONDUCTOR. This device can have approximately 2pF capacitance between the drive and sense circuits.

In the above examples, the DC to DC convertor 27 and the opto-isolator 25 are separate discrete components within the isolator 23. However, it is to be appreciated that an integrated isolator to provide the above described functionality could be used.

In some examples, the coupling capacitance between the amplifier module 5 and the control module 15 (and power source 3) is between 2 to 20 pF.

Fig. 25b is a broader system view of the embodiment of Fig. 25a. This clarifies that the stray input capacitance of the amplifier module is separated from the stray capacitance of the control module. We aim to make the isolation impedance Zi much greater than the amplifier input impedances Zᵢ₁ and Zᵢ₂ (labelled as 14 in Fig. 25a). The co-existence of the stimulator and the amplifier and the problem of their separate ground points is to be noted. In the context of an implantable pulse generator, where the PCB is made to be as small as possible, stray capacitance is a significant problem and so this problem becomes acute. Usually Zᵢ₁ and Zᵢ₂ would be stray capacitances to ground but there could be other components such as amplifier bias.

### The control module 15 (Fig. 24)

The control module 15 includes a microprocessor to control the various components of the implantable pulse generator system 1. As noted above, this can include functioning as a controller to receive sensed information (e.g. from the feedback signal 13') and to provide respective control signals 17 to direct the stimulator module 19 to deliver the stimulation current. In some examples, this closed-loop feedback system includes comparing applied stimulation current to the ECAP (as indicated by the feedback signal) to determine the effectiveness and response of that stimulation current. In response, the control module 15 can modify a control signal 17 to provide a stimulation current that will evoke a neural response closer to a desired or target ECAP.

It is to be appreciated that the control module 15, in some examples, controls other components of the system 1, including one or more of the power source 3, amplifier module 5, and isolator 23.

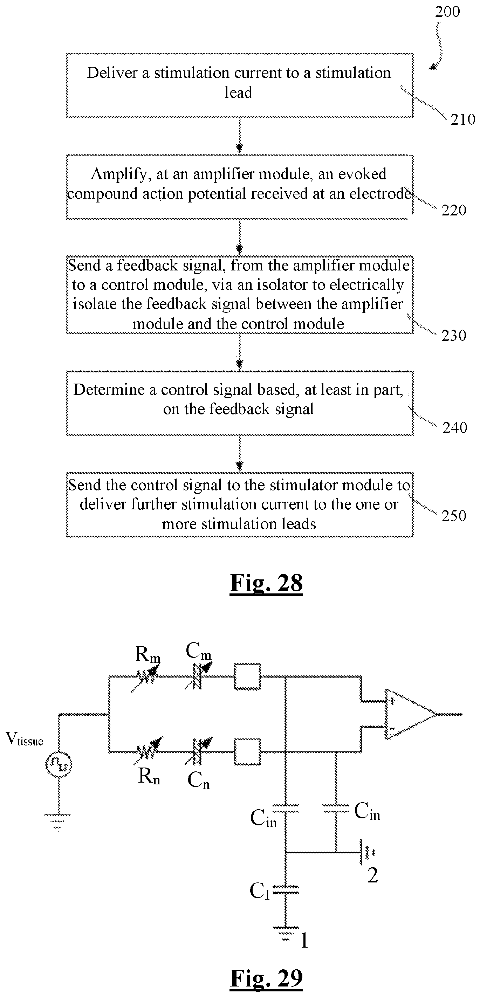

Referring to Fig. 28, there is also provided a method 200 of closed-loop spinal cord stimulation with an implantable pulse generator system 1 controlled by the control module 15. This includes delivering 210 a stimulation current with a stimulator module 19 to one or more one or more stimulation leads 21. The amplifier module 5 amplifies 220 an evoked compound action potential 9 received at one or more measurement electrodes 11 to produce a feedback signal 13. The feedback signal 13 is sent 230 from the amplifier module 5 to a control module 15 via an isolator 23 to electrically isolate the feedback signal 13 between the amplifier module 5 and the control module 15. The control module 15 determines 240 a control signal 17 based, at least in part, on the feedback signal 13. The control signal 17 is sent 250 from the control module 15 to the stimulator module 19 to deliver further stimulation current to the one or more stimulation leads 21.

In some examples the method 200 further includes sending power from a power source to the amplifier module 5 via the isolator 23, or another isolator, to electrically isolate the power source 3 and the amplifier module 5.

In further examples, the method includes grounding the power source 3, control module 15, and stimulator module 19 to the first electrical ground 29 and grounding the amplifier module 5 to the second electrical ground 31. In further examples, the first electrical ground 29 is also grounded to the IPG case 33.

### Stimulator module 19 and power source 3 (Fig. 24)

The stimulator module 19 delivers stimulation current based on the control signal 17 provided by the control module 15. The stimulator module 19 may include current sources and switches to provide the stimulation current as specified by the control signal 17/control module 15. The specified stimulation current may include amplitude, type, timing, shape, and other characteristics. In some examples, the stimulator module 19 may include components of, or is an adaptation of, the stimulator module 1019 described above.

The power source 3 may include a battery. In some examples, the power source 3 is an internal battery housed within the IPG case 33. In other examples, the power source 3 is external to the IPG case 33. In some examples, it is desirable that the power source is grounded to the IPG case 33 which is, at least in part, constructed of an electrically conductive material.

In some examples the power source 3 is a primary (non-rechargeable) battery. In other examples, the power source 3 includes, at least in part, a secondary battery (i.e. a rechargeable battery). In some examples, the power source 3 and related system may include an induction coil to receive energy for powering the IPG and/or recharging the battery.

In the example of Fig. 25b the isolator consists of a DC-to-DC converter and an opto-isolator. The isolator interfaces the power and control signals between the main IPG circuitry and the amplifier-ADC pair. Note that the "ground" for the amplifier (ground 2) is not the same as the "ground" for the main body of the IPG (ground 1).

Separating the two parts of the system leads to Fig. 29 where the isolation impedance *Z_{I}* is assumed to be purely capacitive and represented by *C_{I}*. As mentioned previously, the design problem is that of determining the characteristics of the isolation impedance shown in Fig. 25b as Z_{I} between the two modules.

For example, if we were to assume that *Cᵢₙ* = 100*pF* then to satisfy Equation 19, following standard design methods, *C_{I}* < 30*pF.*

The Murata NKA0303SC optically isolated power supply has an isolation capacitance of 20pF. The capacitance of the MID400 isolated driver from On Semiconductor between the drive and sense circuits is 2pF. These components are small so this could be fit within a standard IPG case and between them provide isolation capacitance of 22pF. Thus it is clear that the isolator providing both power and communications could be designed and built with low coupling between the two sides. These are off-the-shelf items. If lower capacitance is required a custom isolated power supply could be designed.

An alternative embodiment may involve placing the stimulator in the isolation region. This is not the preferred embodiment as the power requirements of the stimulator are higher and more impulsive than that of the amplifier, so the design of the power isolator would be more difficult.

In other aspects of the present technology, which may be combined with some implementations of the previously described aspects of the present technology, it is observed that when considering Equation 3, artefact can be reduced by reducing both electrode impedances *Z*_{*c*2} and *Z*_{*c*1}. Having identified this objective, there are a number of ways by which electrode impedance can be reduced, for the sense electrodes in particular. These include (i) using an electrode surface treatment that makes the electrode more polarizable. Such treatments include coating with titanium nitride, or carbon, or Amplicoat; (ii) increasing the surface area of the electrodes. At a large scale, this includes surface treatment such as cutting grooves in the electrodes (either mechanically or with lasers) and at a small scale this involves surface treatment such as roughening the electrodes e.g. by chemical means such as otonizing; (iii) increasing the size of the electrodes, e.g. by making the electrodes longer. These methods will reduce electrode impedance of the sense electrodes, and so will improve artefact.

It is noted that the split electrode phenomenon (described further in the aforementioned WO2020/082126) can cause artefact particularly on electrodes close to the stimulus electrodes. In contrast, impedance mismatch effects addressed herein can cause artefact on all electrodes. Accordingly reducing electrode impedance as by increasing the electrode size may be particularly applicable to sense electrodes that are outside or only minimally impacted by the stimulation field, in order to decrease net artefact.

Yet another implementation of reducing artefact provides for treating the electrode surface, such as by increasing the sense electrode surface area, or by coating the sense electrode to improve polarizability, and thereby reducing electrode impedance and thus reducing artefact, in combination with relatively minimising a length of the electrode exposed to the stimulation field (the "effective length"), to minimise split electrode effects and thus also improve artefact.

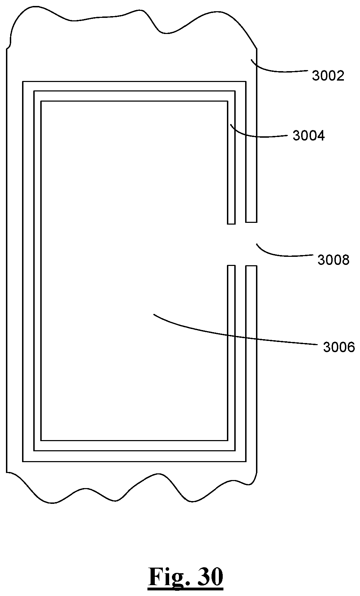

For example, a metal coil or sponge electrode may be enclosed in an insulator, and a small opening may be made in the insulator to communicate with the external field. This could be an annular opening or it could be on only one side of the lead, for a directed recording field. Another option, as shown in Fig. 30, would be to use a hollow ring electrode 3004 coated in insulator 3002, and to pierce a hole 3008 through both the insulator and the ring. This allows for a field gradient to arise inside the enclosed volume 3006, but the small size of the aperture exposes the electrode to almost a point source in the external tissue field, rather than exposing the electrode to a tissue field gradient along the full length of the electrode.

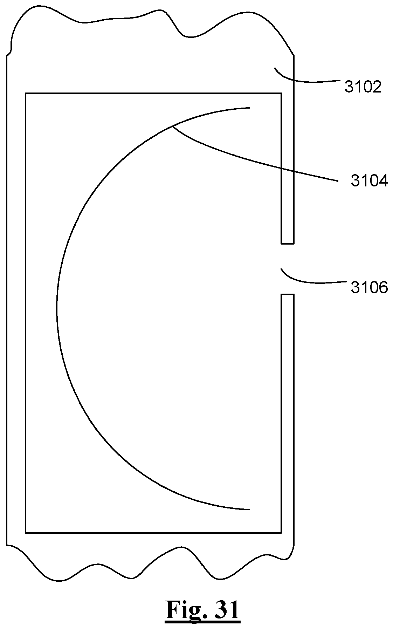

As another example, as shown in **Fig. 31****,** a curved conductor 3104, which includes a metal coil or sponge electrode, may be enclosed in an insulator 3102, and a small opening 3106 may be made in the insulator to permit the conductor 3104 to communicate with the external field. The curved shape of the conductor 3104 enables even distribution of the charge caused by the stimulation field along the conductor and prevents accumulation of electrical charge at the corners. The opening 3106 could be an annular opening or it could be on only one side of the lead, for a directed recording field.

In some implementations, the effective length of an electrode may be decreased by simply decreasing a rostro-caudal dimension of the electrode ("shortening" the electrode as described in the aforementioned WO 2020/082126). Such implementations may, all other things being equal, reduce the current carrying capacity of the electrode, which is not normally desirable in electrode design. For example, for a platinum/iridium electrode a charge density of 1 uC/sq mm is common. For example, a typical epidural electrode has a maximum charge of 12.7 uC over an area of 12.3 sq mm. A typical paddle electrode has an area of 6 sq mm and a maximum charge of 6 uC. However, when shortening of an electrode is combined with surface treatment as described above, artefact may be reduced without sacrificing the current carrying capacity of the electrode. In other words, the longitudinal dimension of the electrode may be decreased to the extent that the surface treatment allows while maintaining current carrying capacity. The artefact-reducing effects of the surface treatment combine synergistically with the those of the decreased effective length of the electrode to reduce artefact by substantially more than either measure taken alone.

Aspects of the present technology thus involve recognising the existence of artefact which arises as a result of mismatched amplifier input impedances, mismatched CPE impedances such as may be caused by uneven tissue growth, and/or interaction between amplifier input impedance and CPE impedance, and based upon this recognition taking steps to reduce or eliminate such artefact. Uneven tissue growth never occurs in a saline bath making this problem particularly difficult to identify, understand or solve. However when equipped with the present technology, it is possible to comply with Equation 19 and design to this criterion to achieve the desired outcome.

The claimed electronic functionality can be implemented by discrete components mounted on a printed circuit board, or by a combination of integrated circuits, or by an application-specific integrated circuit (ASIC).

## Claims

1. A device (100) for recording evoked neural responses, the device comprising:
a plurality of electrodes (150) including one or more stimulus electrodes, a first sense electrode, and a second sense electrode;
a stimulus source (124) for providing a stimulus to be delivered via the one or more stimulus electrodes to a neural pathway in order to evoke a compound action potential on the neural pathway;
measurement circuitry for processing a signal sensed at the first sense electrode and second sense electrode, the sensed signal comprising the evoked compound action potential; and **characterised by**
impedance compensation means configured to compensate for an impedance difference, the impedance difference being a difference between an impedance associated with the first sense electrode and an impedance associated with the second sense electrode.

2. The device (100) of claim 1, wherein the impedance compensation means comprises one or more compensating impedances connected so as to compensate for the impedance difference.

3. The device (100) of claim 2, wherein the one or more compensating impedances have fixed impedance values.

4. The device (100) of claim 2, wherein the one or more compensating impedances have configurable impedance values, preferably wherein the configurable impedance values are configurable under control of a feedback loop that operates based on artefact measured in the sensed signal.

5. The device (100) of claim 1, wherein the impedance compensation means comprises an electrical shield positioned around or substantially around at least one conductor conveying a potential from a sense electrode to the measurement circuitry.

6. The device (100) of claim 5, wherein the electrical shield is driven, preferably wherein the electrical shield is driven with a voltage derived from one or more unused electrodes of the plurality of electrodes.

7. The device (100) of claim 1, wherein the measurement circuitry comprises an amplifier module (128s) configured to generate a feedback signal from the sensed signal.

8. The device (100) of claim 7, wherein the device further comprises a control module to receive the feedback signal and to send a control signal to the stimulus source.

9. The device (100) of claim 8, wherein the impedance compensation means comprises an isolator configured to electrically isolate the measurement circuitry from the control module, preferably wherein the isolator is configured such that a coupling capacitance between the amplifier module and the control module is lower than or equal to 20 pF.

10. The device (100) of any one of claims 8 to 9, wherein the device further comprises a power source configured to provide power to one or more of the amplifier module, the control module, and the stimulus source, preferably wherein the impedance compensation means comprises an isolator configured to electrically isolate the power source from the amplifier module.

11. The device (100) of claim 1, wherein the impedance compensation means comprises an input capacitance of the measurement circuitry that is small enough that artefact induced by the impedance difference in the sensed signal is below a predetermined limit at a predetermined time after the delivered stimulus.

12. The device (100) of claim 1, wherein the impedance compensation means comprises a surface treatment to one or both of the sense electrodes, wherein the or each surface treatment is configured to reduce the impedance associated with the corresponding sense electrode, preferably wherein the surface treatment is a coating configured to increase the polarizability of the sense electrode.

13. The device (100) of claim 12, wherein the surface treatment is configured to increase the surface area of the electrode, preferably wherein the surface treatment comprises a grooving or a roughening.

14. The device (100) of any one of claims 12 to 13, wherein the surface treatment may comprise minimising a length of the electrode that is exposed to a stimulation field, preferably wherein minimising a length comprises enclosing the electrode in an insulator with a small opening in the insulator.

15. A method for recording evoked neural responses, the method comprising:
delivering, by a stimulus source, a stimulus via one or more stimulus electrodes of a plurality of electrodes to a neural pathway in order to evoke a compound action potential on the neural pathway;
processing, with measurement circuitry, a signal sensed at a first sense electrode and a second sense electrode of the plurality of electrodes, the sensed signal comprising the evoked compound action potential; and **characterised by**
compensating for an impedance difference, the impedance difference being a difference between an impedance associated with the first sense electrode and an impedance associated with the second sense electrode.

## Patentansprüche

1. Vorrichtung (100) zum Aufzeichnen evozierter neuronaler Reaktionen, wobei die Vorrichtung aufweist:
eine Mehrzahl von Elektroden (150), die eine oder mehrere Reizelektroden, eine erste Sensorelektrode und eine zweite Sensorelektrode aufweist;
eine Reizquelle (124) zum Bereitstellen eines Reizes, der über die eine oder die mehreren Reizelektroden an eine Nervenbahn abzugeben ist, um ein Summenaktionspotential an der Nervenbahn zu evozieren;
eine Messschaltungsanordnung zur Verarbeitung eines an der ersten Sensorelektrode und der zweiten Sensorelektrode erfassten Signals, wobei das erfasste Signal das evozierte Summenaktionspotential aufweist; und **gekennzeichnet durch**
eine Impedanzkompensationseinrichtung, die dazu ausgelegt ist, eine Impedanzdifferenz zu kompensieren, wobei die Impedanzdifferenz eine Differenz zwischen einer der ersten Sensorelektrode zugeordneten Impedanz und einer der zweiten Sensorelektrode zugeordneten Impedanz ist.

2. Vorrichtung (100) nach Anspruch 1, wobei die Impedanzkompensationseinrichtung eine oder mehrere Kompensationsimpedanzen aufweist, die so verbunden sind, dass sie die Impedanzdifferenz kompensieren.

3. Vorrichtung (100) nach Anspruch 2, wobei die eine oder die mehreren Kompensationsimpedanzen feste Impedanzwerte aufweisen.

4. Vorrichtung (100) nach Anspruch 2, wobei die eine oder die mehreren Kompensationsimpedanzen konfigurierbare Impedanzwerte aufweisen, wobei die konfigurierbaren Impedanzwerte vorzugsweise unter der Steuerung einer Rückkopplungsschleife konfigurierbar sind, die auf Grundlage eines in dem erfassten Signal gemessenen Artefakts arbeitet.

5. Vorrichtung (100) nach Anspruch 1, wobei die Impedanzkompensationseinrichtung eine elektrische Abschirmung aufweist, die um, oder im Wesentlichen um, mindestens einen Leiter herum angeordnet ist, der ein Potential von einer Sensorelektrode zu der Messschaltungsanordnung überträgt.

6. Vorrichtung (100) nach Anspruch 5, wobei die elektrische Abschirmung angesteuert wird, wobei die elektrische Abschirmung vorzugsweise mit einer Spannung angesteuert wird, die von einer oder mehreren ungenutzten Elektroden der Mehrzahl von Elektroden abgeleitet wird.

7. Vorrichtung (100) nach Anspruch 1, wobei die Messschaltungsanordnung ein Verstärkermodul (128s) aufweist, das dazu ausgelegt ist, aus dem erfassten Signal ein Rückkopplungssignal zu erzeugen.

8. Vorrichtung (100) nach Anspruch 7, wobei die Vorrichtung ferner ein Steuermodul aufweist, um das Rückkopplungssignal zu empfangen und ein Steuersignal an die Reizquelle zu senden.

9. Vorrichtung (100) nach Anspruch 8, wobei die Impedanzkompensationseinrichtung einen Isolator aufweist, der dazu ausgelegt ist, die Messschaltungsanordnung elektrisch von dem Steuermodul zu isolieren, wobei der Isolator vorzugsweise derart ausgelegt ist, dass eine Kopplungskapazität zwischen dem Verstärkermodul und dem Steuermodul kleiner oder gleich 20 pF ist.

10. Vorrichtung (100) nach einem der Ansprüche 8 bis 9, wobei die Vorrichtung ferner eine Energiequelle aufweist, die dazu ausgelegt ist, dem Verstärkermodul und/oder dem Steuermodul und/oder der Reizquelle Energie bereitzustellen, wobei die Impedanzkompensationseinrichtung vorzugsweise einen Isolator aufweist, der dazu ausgelegt ist, die Energiequelle elektrisch von dem Verstärkermodul zu isolieren.

11. Vorrichtung (100) nach Anspruch 1, wobei die Impedanzkompensationseinrichtung eine Eingangskapazität der Messschaltungsanordnung aufweist, die klein genug ist, dass ein durch die Impedanzdifferenz in dem erfassten Signal induziertes Artefakt zu einer vorbestimmten Zeit nach dem abgegebenen Reiz unter einer vorbestimmten Grenze liegt.

12. Vorrichtung (100) nach Anspruch 1, wobei die Impedanzkompensationseinrichtung eine Oberflächenbehandlung an einer oder beiden Sensorelektroden aufweist, wobei die oder jede Oberflächenbehandlung dazu ausgelegt ist, die der entsprechenden Sensorelektrode zugeordnete Impedanz zu reduzieren, wobei die Oberflächenbehandlung vorzugsweise eine Beschichtung ist, die dazu ausgelegt ist, die Polarisierbarkeit der Sensorelektrode zu erhöhen.

13. Vorrichtung (100) nach Anspruch 12, wobei die Oberflächenbehandlung dazu ausgelegt ist, den Oberflächenbereich der Elektrode zu vergrößern, wobei die Oberflächenbehandlung vorzugsweise Rillen oder eine Aufrauhung aufweist.

14. Vorrichtung (100) nach einem der Ansprüche 12 bis 13, wobei die Oberflächenbehandlung das Minimieren einer Länge der Elektrode aufweisen kann, die einem Stimulationsfeld ausgesetzt ist, wobei das Minimieren einer Länge vorzugsweise das Umgeben der Elektrode mit einem Isolator mit einer kleinen Öffnung in dem Isolator aufweist.

15. Verfahren zum Aufzeichnen evozierter neuronaler Reaktionen, wobei das Verfahren aufweist:
das Abgeben eines Reizes von einer Reizquelle über eine oder mehrere Reizelektroden von einer Mehrzahl von Elektroden an eine Nervenbahn, um ein Summenaktionspotential an der Nervenbahn zu evozieren;
Verarbeiten eines an einer ersten Sensorelektrode und einer zweiten Sensorelektrode der Mehrzahl von Elektroden erfassten Signals mit einer Messschaltungsanordnung, wobei das erfasste Signal das evozierte Summenaktionspotential aufweist; und **gekennzeichnet durch**
Kompensieren einer Impedanzdifferenz, wobei die Impedanzdifferenz eine Differenz zwischen einer der ersten Sensorelektrode zugeordneten Impedanz und einer der zweiten Sensorelektrode zugeordneten Impedanz ist.

## Revendications

1. Dispositif (100) d'enregistrement de réponses neuronales évoquées, le dispositif comprenant :
une pluralité d'électrodes (150) comprenant une ou plusieurs électrodes de stimulus, une première électrode de détection et une seconde électrode de détection ;
une source de stimulus (124) pour fournir un stimulus à délivrer par l'intermédiaire des une ou plusieurs électrodes de stimulus à une voie neuronale afin d'évoquer un potentiel d'action de composé sur la voie neuronale ;
une circuiterie de mesure pour le traitement d'un signal détecté au niveau de la première électrode de détection et de la seconde électrode de détection, le signal détecté comprenant le potentiel d'action de composé évoqué ; et **caractérisé par**
un moyen de compensation d'impédance configuré pour compenser une différence d'impédance, la différence d'impédance étant une différence entre une impédance associée à la première électrode de détection et une impédance associée à la seconde électrode de détection.

2. Dispositif (100) selon la revendication 1, dans lequel le moyen de compensation d'impédance comprend une ou plusieurs impédances de compensation connectées de manière à compenser la différence d'impédance.

3. Dispositif (100) selon la revendication 2, dans lequel une ou plusieurs impédances de compensation ont des valeurs d'impédance fixes.

4. Dispositif (100) selon la revendication 2, dans lequel une ou plusieurs impédances de compensation ont des valeurs d'impédance configurables, de préférence dans lequel les valeurs d'impédance configurables sont configurables sous la commande d'une boucle de rétroaction qui fonctionne en fonction d'un artefact mesuré dans le signal détecté.

5. Dispositif (100) selon la revendication 1, dans lequel le moyen de compensation d'impédance comprend un blindage électrique positionné autour ou sensiblement autour d'au moins un conducteur transmettant un potentiel depuis une électrode de détection jusqu'à la circuiterie de mesure.

6. Dispositif (100) selon la revendication 5, dans lequel le blindage électrique est commandé, de préférence dans lequel le blindage électrique est commandé par une tension dérivée d'une ou plusieurs électrodes inutilisées de la pluralité d'électrodes.

7. Dispositif (100) selon la revendication 1, dans lequel la circuiterie de mesure comprend un module amplificateur (128s) configuré pour générer un signal de rétroaction à partir du signal détecté.

8. Dispositif (100) selon la revendication 7, dans lequel le dispositif comprend en outre un module de commande destiné à recevoir le signal de rétroaction et à envoyer un signal de commande à la source de stimulus.

9. Dispositif (100) selon la revendication 8, dans lequel le moyen de compensation d'impédance comprend un isolateur configuré pour isoler électriquement la circuiterie de mesure du module de commande, de préférence dans lequel l'isolateur est configuré de telle sorte qu'une capacité de couplage entre le module amplificateur et le module de commande soit inférieure ou égale à 20 pF.

10. Dispositif (100) selon l'une quelconque des revendications 8 à 9, dans lequel le dispositif comprend en outre une source d'énergie configurée pour fournir de l'énergie à un ou plusieurs parmi le module amplificateur, le module de commande et la source de stimulus, de préférence dans lequel le moyen de compensation d'impédance comprend un isolateur configuré pour isoler électriquement la source d'énergie du module amplificateur.

11. Dispositif (100) selon la revendication 1, dans lequel le moyen de compensation d'impédance comprend une capacité d'entrée de la circuiterie de mesure suffisamment petite pour que l'artefact induit par la différence d'impédance dans le signal détecté soit inférieur à une limite prédéterminée à un moment prédéterminé après le stimulus délivré.

12. Dispositif (100) selon la revendication 1, dans lequel le moyen de compensation d'impédance comprend un traitement de surface appliqué à l'une ou aux deux électrodes de détection, dans lequel le ou chaque traitement de surface est configuré pour réduire l'impédance associée à l'électrode de détection correspondante, de préférence dans lequel le traitement de surface est un revêtement configuré pour augmenter la polarisabilité de l'électrode de détection.

13. Dispositif (100) de la revendication 12, dans lequel le traitement de surface est configuré pour augmenter la surface de l'électrode, de préférence dans lequel le traitement de surface comprend un rainurage ou une rugosification.

14. Dispositif (100) selon l'une quelconque des revendications 12 à 13, dans lequel le traitement de surface peut comprendre la minimisation d'une longueur de l'électrode exposée à un champ de stimulation, de préférence dans lequel la minimisation d'une longueur comprend l'enfermement de l'électrode dans un isolateur avec une petite ouverture dans l'isolateur.

15. Procédé d'enregistrement de réponses neuronales évoquées, le procédé comprenant :
le fait de délivrer, par une source de stimulus, un stimulus par l'intermédiaire d'une ou plusieurs électrodes de stimulus d'une pluralité d'électrodes à une voie neuronale afin d'évoquer un potentiel d'action de composé sur la voie neuronale ;
le traitement, avec une circuiterie de mesure, d'un signal détecté à une première électrode de détection et une seconde électrode de détection de la pluralité d'électrodes, le signal détecté comprenant le potentiel d'action de composé évoqué ; et **caractérisé par**
la compensation d'une différence d'impédance, la différence d'impédance étant une différence entre une impédance associée à la première électrode de détection et une impédance associée à la seconde électrode de détection.
